# EUROPEAN PATENT APPLICATION

(11) **EP 1 493 809 A1**
(43) Date of publication of application: **05.01.2005**
(21) Application number: 03745993.0
(22) Date of filing: 10.04.2003
(51) Int. Cl.: C12N 15/09, C12N 1/21, C12N 9/78, C12N 9/86, C12N 9/90

(54) **RECOMBINANT DNA HAVING HYDANTOINASE GENE AND CARBAMYLASE GENE AND PROCESS FOR PRODUCING AMINO ACID**

(30) Priority: 10.04.2002 JP 2002108550
(71) Applicant: Ajinomoto Co., Inc., Tokyo 104-8315 (JP)
(72) Inventor: KIRA, Ikuo, Kawasaki-shi, Kanagawa 210-8681 (JP); TAKENAKA, Yasuhiro, Kawasaki-shi, Kanagawa 210-8681 (JP); NOZAKI, Hiroyuki, Kawasaki-shi, Kanagawa 210-8681 (JP); WATANABE, Kunihiko, Kawasaki-shi, Kanagawa 210-8681 (JP)
(74) Representative: Strehl Schübel-Hopf & Partner
(86) International application number: PCT/JP2003/004553
(87) International publication number: WO 2003/085108

(57) **Abstract**

The object of the present invention is to prepare a recombinant DNA efficiently co-expressing genes of enzymes such as HHase, and to produce an amino acid efficiently from hydantoin by utilizing such a recombinant DNA. The present invention provides a recombinant DNA having base sequences encoding the following gene regions (I) and (II) incorporated therein in a predetermined direction and in a predetermined order to produce an amino acid efficiently:
(I) a gene region containing a base sequence encoding a hydantoinase gene and a trp promoter sequence located upstream of the base sequence encoding the hydantoinase gene regulating expression of the hydantoinase gene, and
(II) a gene region containing a base sequence encoding a carbamylase gene and a trp promoter sequence located upstream of the base sequence encoding the carbamylase gene regulating expression of the carbamylase gene.

## Description

### TECHNICAL FIELD

The present invention relates to a recombinant DNA and a process for producing an amino acid by using the same, and in particular to a recombinant DNA co-expressing a hydantoinase gene and a carbamylase gene efficiently and a process for producing an amino acid with high productivity by using the same.

### BACKGROUND ART

One of known methods for producing an amino acid by use of an enzyme is asymmetric decomposition of a 5-substituted hydantoin compound. The 5-substituted hydantoin compound may be synthesized chemically at a low cost as the starting material, and may be decomposed to be an optically active amino acid. This method for producing the optically active amino acids from the 5-substituted hydantoin compounds is important in the production of pharmaceutical preparations, chemical products, food additives, etc.

As one of methods for producing the optically active amino acid from the 5-substituted hydantoin compound, there is a method using the following enzymes (A) and (B):
(A) An enzyme that catalyzes hydrolytic reaction of the 5-substituted hydantoin compound to form an N-carbamylamino acid; this enzyme is called hydantoinase (also known as hydantoin hydrolase; the enzyme may be referred to hereinafter as "HHase").
(B) An enzyme that catalyzes hydrolytic reaction of the obtained N-carbamylamino acid to form an amino acid; this enzyme is called carbamylase (also known as N-carbamylamino acid hydrolase; the enzyme may be referred to hereinafter as "CHase").

If at least one of the enzymes (A) HHase and (B) CHase is optically selective, an optically active amino acid may be produced from the 5-substituted hydantoin compound.

Use of the aforementioned enzymes may be performed by introducing genes encoding HHase and CHase into a host such as *Escherichia coli* to prepare a transformant, and then culturing this transformant, whereby an amino acid may be formed from the 5-substituted hydantoin and accumulated in the medium. A method for producing an amino acid by utilizing a recombinant DNA is reported in, for example, WO 01/23582 and WO 05/8449. In the techniques described therein, a rhamnose promoter is used as a promoter for expression of HHase, etc.

### DISCLOSURE OF THE INVENTION

There have been developed a variety of practical techniques as to ligation of an extraneous gene with a recombinant vector and introduction of such a vector into a cell such as a microorganism for the expression of the genes in the cell. Examples of the techniques directed to amino acid production may include those described in the above two literatures. The techniques described in the literatures utilize a rhamnose promoter. It is necessary to add rhamnose as an inducer at a predetermined time for exerting the function of the rhamnose promoter. Upon adding rhamnose, it is very important to adjust the time for addition. Industrial production of an amino acid using such techniques thus requires an additional step for adding the inducer, the timing of which has to be carefully regulated. Therefore, the production steps thereof are undesirably complicated.

Lac promoter is one of the well-known promoters. In the industrial mass production of an amino acid with a transformed microorganism, the microorganisms should be maintained at a high density. In order to maintain such a high-density in the culture, glucose may have to be added as a nutrient source. However, addition of glucose leads to suppression of the lac promoter, and thus use of the lac promoter may not be preferable in the industrial production of an amino acid.

Accordingly, it is necessary to chose suitable combination of the promoters and host, etc. depending on specific situations. What is possible at the laboratory level is not always industrially advantageous. Employment of a promoter and the like that are known to be capable of enhancing expression level does not always result in satisfactory production. Further, when a plurality of enzymes are to be expressed as described above, the co-expression of the genes should be performed in a well-balanced manner to meet the final objectives.

The present invention has been made in the light of the aforementioned viewpoints. The object of the present invention is to provide a recombinant DNAthat is capable of efficiently co-expressing genes of enzymes such as HHase, etc. and to efficiency produce an amino add from hydantoin by utilizing such a recombinant DNA.

The present inventors have made extensive studies for obtaining a recombinant DNA capable of efficiently co-expressing HHase and CHase genes. As a result, they have found out that, among a large number of promoters, a trp promoter is capable of efficiently expressing HHase, CHase and hydantoin racemase (which may be referred to hereinafter as "HRase") and suitable in the industrial production of the amino acid. The present inventors have also found out that the activity of each enzyme is significantly affected by the arrangement of the HHase and CHase genes upon introducing them into the recombinant DNA, such as the direction of the genes in a recombinant DNA and the order of insertion of the genes, thereby completing the present invention.

That is, the present invention is as follows:
[1] A recombinant DNA comprising a base sequence encoding the following gene region (I), and another base sequence encoding the following gene region (II):
   (I) a gene region containing a base sequence encoding a hydantoinase gene, and a trp promoter sequence located upstream of the base sequence encoding the hydantoinase gene, said trp promoter regulating expression of the hydantoinase gene;
   (II) a gene region containing a base sequence encoding a carbamylase gene, and a trp promoter sequence located upstream of the base sequence encoding the carbamylase gene, said trp promoter regulating expression of the carbamylase gene.
[2] The recombinant DNA according to the above-mentioned [1], wherein the transcriptional direction of the base sequence encoding the gene region (I) is the same as that of the base sequence encoding the gene region (II), and wherein the base sequence encoding the gene region (I) and the base sequence encoding the gene region (II) are disposed in this order along the transcriptional direction.
[3] The recombinant DNA according to the above-mentioned [1], wherein the transcriptional direction of the base sequence encoding the gene region (I) is in the reverse direction of the base sequence encoding the gene region (II).
[4] The recombinant DNA according to the above-mentioned [1], wherein the transcriptional direction of the base sequence encoding the gene region (I) is the same as that of the base sequence encoding the gene region (II), and wherein the base sequence encoding the gene region (II) and the base sequence encoding the gene region (I) are disposed in this order along the transcriptional direction.
[5] The recombinant DNA according to any one of the above-mentioned [1] to [4], wherein the recombinant DNA is a plasmid.
[6] A transformed cell transformed with the recombinant DNA according to any one of the above-mentioned [1] to [5].
[7] A transformed cell transformed with:
   the recombinant DNA according to any one of the above-mentioned [1] to [5]; and
   a recombinant DNA having a base sequence encoding a hydantoin racemase gene and a trp promoter sequence located upstream of the hydantoin racemase gene, said trp promoter regulating expression of the hydantoin racemase gene.
[8] The transformed cell according to the above-mentioned [7], wherein the recombinant DNA according to any one of the above-mentioned [1] to [5] is a low-copy recombinant DNA, and the recombinant DNA having the base sequence encoding the hydantoin racemase gene and the trp promoter sequence located upstream of the hydantoin racemase gene for regulating expression of the hydantoin racemase gene is a high-copy recombinant DNA.
[9] The transformed cell according to any one of the above-mentioned [6] to [8], wherein the host of the recombinant DNA is *Escherichia coli*.
[10] A process for producing a mixture of proteins comprising the step of culturing the transformed cell according to the above-mentioned [6] in a medium, to accumulate a protein having a hydantoinase activity and a protein having a carbamylase activity in the medium and/or the transformed cell.
[11] A process for producing a mixture of proteins comprising the step of culturing the transformed cell according to the above-mentioned [7] in a medium, to accumulate a protein having a hydantoinase activity, a protein having a carbamylase activity and a protein having a hydantoin racemase activity in the medium and/or the transformed cell.
[12] A process for producing an amino acid comprising the step of culturing the transformed cell according to any one of the above-mentioned [6] to [9] in a medium to give a culture, and the step of mixing 5-substituted hydantoin with the culture to form the amino acid.

### BRIEF DESCRIPTION OF THE DRAWINGS

Fig. 1 is a diagram showing the location and direction of inserted HHase and CHase in a plasmid.
Fig. 2 is a diagram showing trp promoter cassettes 1 and 2.
Fig. 3 is a diagram showing the construction of pTrpHfCf.
Fig. 4 is a diagram showing the construction of pTrpCrHf.
Fig. 5 is a diagram showing the construction of pTrpHrCr.
Fig. 6 is a diagram showing the construction of pTrp4R.
Fig. 7 is a diagram showing the construction of pTrp8CH.

### BEST MODE FOR CARRYING OUT THE INVENTION

The present invention will be described hereinbelow in detail with an order of "1. Recombinant DNA, etc.", "2. Process for producing the enzyme protein", and "3. Process for producing an amino acid."

### 1. Recombinant DNA, etc.

The recombinant DNA of the present invention contains base sequences encoding the following gene regions (I) and (II):
(I) A gene region containing a base sequence encoding an HHase gene and a trp promoter sequence located upstream of the HHase gene sequence for regulating expression of the HHase gene (this gene region may be referred to hereinafter as "trp+HHase". In the Drawings, the trp promoter is referred to as "Ptrp".)
(II) A gene region containing a base sequence encoding a CHase gene and a trp promoter sequence located upstream of the CHase gene sequence for regulating expression of the CHase gene (this gene region may be referred to hereinafter as "trp+CHase").

That is, the recombinant DNA of the present invention has the trp promoter as a promoter for expressing each of HHase and CHase. The trp promoter is found in e.g., *Escherichia coli* and located upstream of genes belonging to a gene group encoding some enzymes involved in biosynthesis of tryptophan. In the present invention, a known trp promoter may be used. The trp promoter is commercially available in the form of a trp promoter-containing cloning vector or a cell containing such a vector. Employment of the trp promoter results in a sufficient expression of HHase and CHase. The trp promoter does not require addition of an inducer such as rhamnose that is requisite when a rhamnose promoter is employed. Accordingly, steps and treatments accompanying addition of the inducer can be obviated in the present process for producing the amino acid. Further, the activity of the trp promoter is not suppressed by glucose, and thus the expression levels of the enzymes are not suppressed even if glucose is used in a high-density culture of the transformed microorganisms in the production of the amino acid on an industrial scale.

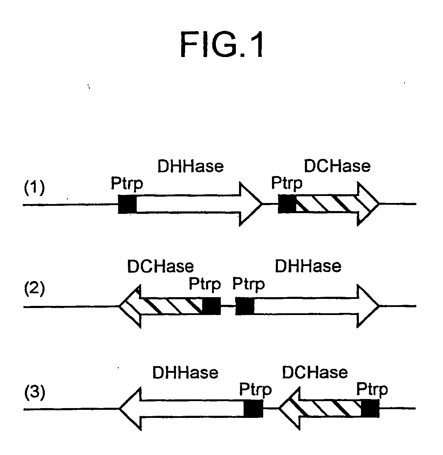

There are three embodiments in terms of the directions and order of insertion of the DNA having the base sequence encoding HHase (also referred to hereinafter as "DNA encoding HHase") and the DNA having the base sequence encoding CHase (also referred to hereinafter as "DNA encoding CHase"), into a recombinant DNA such as a vector.
(First embodiment) The base sequence encoding the gene region (I) and the base sequence encoding the gene region (II) are inserted in the same transcriptional direction, and in the order of the base sequence encoding the gene region (I) and then the base sequence encoding the gene region (II), along the transcriptional direction (Fig. 1(1)). That is, in Fig. 1(1), the transcriptional directions of both the "trp+HHase" gene region and "trp+CHase" gene region are from left to right; and the "trp+HHase" gene region and "trp+CHase" gene region are disposed in this order from left to right In Fig. 1, DHHase and DCHase that are specific for D-isomer substrates are employed as the examples of HHase and CHase, respectively.
(Second embodiment) The base sequence encoding the gene region (I) and the base sequence encoding the gene region (II) are inserted such that the transcriptional directions thereof are opposed from each other (Fig. 1(2)). That is, in Fig. 1(2), the transcriptional direction of the "trp+CHase" gene region is from right to left, while the transcriptional direction of the "trp+HHase" gene region is from left to right.
(Third embodiment) The base sequence encoding the gene region (I) and the base sequence encoding the gene region (II) are inserted in the same transcriptional direction, and in the order of the base sequence encoding the gene region (II) and then the base sequence encoding the gene region (I), along the transcriptional direction (Fig. 1 (3)). That is, the transcriptional directions of both the "trp+HHase" gene region and "trp+CHase" gene region are from right to left, and the "trp+CHase" gene region and "trp+HHase" gene region are disposed in this order from right to left.

In the examples shown in Fig. 1, "Ptrp" refers to a trp promoter. "DHHase" is HHase having a D-hydantoinase activity, and "DCHase" is CHase having a D-carbamylase activity. Each arrow means that transcription proceeds in the direction of that arrow.

Among these three embodiments, the third embodiment (Fig. 1 (3)) results in the highest co-expression of HHase and CHase. That is, the third embodiment may achieve an efficient, the best-balanced co-expression of the two sorts of the enzyme genes incorporated into one recombinant. The third embodiment is thus particularly preferable in the industrial production of the amino acid on a large scale.

One of possible options for expressing two or more sorts of enzymes in a transformant may be preparation of a plurality of recombinant DNAs each containing only one sort of genes to be expressed, which are then introduced into a host. However, such a transformant may result in an imbalanced expression of the genes due to difference in copy numbers in the host and disappearance of the recombinant DNA. In the present invention, however, two enzyme genes are integrated in one recombinant DNA, by which these genes may be efficiently co-expressed, resulting in the reduced possibility of the imbalance in the expression level of HHase and CHase due to the division and proliferation of the host.

In the production of the amino acids with HHase and CHase, it is desirable that both the enzymes are expressed at high levels. However, in terms of the comparison between HHase and CHase in the reaction system, a relatively higher activity of CHase is desired for efficient production of the amino acid. When the two genes encoding HHase and CHase are integrated in one recombinant, it is desirable that their expression occurs with a suitable balance for the industrial production of the amino acid. The third embodiment is the best in this respect.

The DNAs encoding HHase and CHase, which are to be integrated in the recombinant DNA of the present invention, may be those known in the art HHase may be a protein having a hydantoinase activity, and CHase may be a protein having a carbamylase activity. An optically active amino acid, i.e. either L-amino acid or D-amino acid, may be selectively produced by employing an optically selective enzyme or enzymes as one or more of HHase and CHase. The optical selectivity of the enzyme means that the enzyme specifically uses either L- or D-amino acid as the substrate upon catalyzing the reaction.

HHase for the optically selective hydrolysis of the 5-substituted hydantoin compound may be obtained in accordance with the following manner. As an example of DHHase for the production of N-carbamyl-D-amino acid, it is known that a thermostable enzyme having such an activity exists in microorganisms of genus *Bacillus*. For example, HHase or an HHase-containing fraction may be prepared from *Bacillus stearothermophilus* ATCC 31195, etc. (Appl. Microbiol. Biotechnol. Vol.43 PP. 270, 1995). ATCC 31195 is available from American Type Culture Collection (Address: 12301, Parklawn Drive, Rockville, Maryland 20852, United States of America). It is also known that LHHase specific to L-isomers of the hydantoin compound is present in e.g. *Bacillus* sp. AJ 12299 (Japanese Patent Application Laid-open No. S63-24894 Publication). *Bacillus* sp. AJ 12299 is a microorganism deposited on July 5,1986 under FERM-P8837 with the National Institute of Bioscience and Human-Technology, the Agency of Industrial Science of Technology, the Ministry of International Trade and Industry, JP and transferred on June 27, 2001 as FERM BP-7646 under the Budapest Treaty to International Patent Organism Depositary (IPOD), National Institute of Advanced Industrial Science and Technology (AIST) (Chuo No. 6, Higashi 1-1-1, Tsukuba City, Ibaraki Pref., JP).

It is known that HHase having no optical selectivity is present in e.g. *Arthrobacter aurescens*, as well as *Microbacterium liquefaciens* AJ 3912 (J. Biotechnol. 61, 1 (1998)).

It is known that CHase specifically hydrolyzing the D-isomer of the N-carbamylamino acid is present in, e.g. *Pseudomonas* sp. AJ 11220 (Japanese Patent Application No. S56-003034 Publication). As a result of re-identification, it has been revealed that *Pseudomonas* sp. AJ 11220 in fact belongs to *Agrobacterium* sp. *Agrobacterium* sp. AJ 11220 is a microorganism deposited on December 20,1977, as FERM-P4347 with the National Institute of Bioscience and Human-Technology, the Agency of Industrial Science of Technology, the Ministry of International Trade and Industry, JP, and transferred on June 27, 2001 as FERM BP-7645 under the Budapest Treaty to International Patent Organism Depositary (IPOD), National Institute of Advanced Industrial Science and Technology (AIST). It is known that CHase selectively hydrolyzing the L-isomer of N-carbamylamino acid is present in, e.g. *Flavobacterium* sp. AJ 3912 (Japanese Patent Application No. S56-008749 Publication) and *Bacillus* sp. AJ 12299. As mentioned above, *Flavobacterium* sp. AJ 3912 is now classified into *Microbacterium liquefaciens* AJ 3912 (FERM-P3133), and the microorganism was deposited on June 27,1975, as FERM-P3133 with the National Institute of Bioscience and Human-Technology, the Agency of Industrial Science of Technology, the Ministry of International Trade and Industcy, JP, and transferred on June 27, 2001 as FERM BP-7643 under the Budapest Treaty to International Patent Organism Depositary (IPOD), National Institute of Advanced Industrial Science and Technology (AIST). *Bacillus* sp. AJ 12299 was deposited on July 5, 1986, as FERM-P8837 with the National Institute of Bioscience and Human-Technology, the Agency of Industrial Science of Technology, the Ministry of International Trade and Industry, JP, and transferred on June 27, 2001 as FERM BP-7646 under the Budapest Treaty to International Patent Organism Depositary (IPOD), National Institute of Advanced Industrial Science and Technology (AIST).

In the production of the amino acid by the recombinant DNA of the present invention, examples of the preferable combinations of the DNAs encoding HHase and CHase to be incorporated into the recombinant DNA may include any combination of HHase and CHase each selected from the following. The combination selected from the following will result in the selective production of a D-amino add.

The preferable DNA encoding HHase may be selected from the following (i) to (iv):
(i) a DNA having the base sequence of SEQ ID NO: 1 in the Sequence Listing,
(ii) a DNA having a base sequence that hybridizes under stringent conditions with a DNA consisting of a base sequence complementary to the base sequence of SEQ ID NO:1 in the Sequence Listing,
(iii) a DNA encoding an amino acid sequence of SEQ ID NO:2 in the Sequence Listing, and
(iv) a DNA encoding an amino acid sequence wherein in the amino acid sequence of SEQ ID NO:2 in the Sequence Listing, one or several amino acid residues are replaced, deleted, inserted, added or inverted.

The preferable DNA encoding CHase may be selected from the following (v) to (viii):
(v) a DNA having the base sequence of SEQ ID NO:3 in the Sequence Listing,
(vi) a DNA having a base sequence that hybridizes under stringent conditions with a DNA consisting of a base sequence complementary to the base sequence of SEQ ID NO:3 in the Sequence Listing,
(vii) a DNA encoding an amino acid sequence of SEQ ID NO:4 in the Sequence Listing, and
(viii) a DNA encoding an amino acid sequence wherein in the amino acid sequence of SEQ ID NO:4 in the Sequence Listing, one or several amino acid residues are replaced, deleted, inserted, added or inverted.

As used herein, the "stringent conditions" refer to those conditions under which a specific hybrid is formed whereas an unspecific hybrid is not formed. Although it is difficult to precisely define these conditions with numerals, examples thereof may be the conditions under which DNA molecules having higher homology, e.g. preferably not less than 50 percent, more preferably not less than 80 percent, still more preferably not less than 90 percent homology, hybridize with each other while DNA molecules having lower homology do not hybridize with each other, and the conditions under which hybridization occurs under usual washing conditions in Southern hybridization, that is, at a salt concentration corresponding to 1 ×SSC and 0.1 percent SDS at 60°C, preferably 0.1×SSC and 0.1 percent SDS at 60°C, more preferably 0.1×SSC and 0.1 percent SDS at 65°C.

The term "several" means a number which results in no substantial deterioration of the steric structure of a protein of amino acid residues or the activity of DHHase or DCHase, and is typically 2 to 50, preferably 2 to 30, and more preferably 2 to 10.

The combination of DHHase selected from the aforementioned (i) to (iv) and DCHase selected from the aforementioned (v) to (viii) is particularly advantageous for achieving a necessary enzyme activity for the industrial production of the D-amino acid on a large scale.

The DNAs in SEQ ID NOS:1 to 3 in the Sequence Listing were isolated from chromosomal DNA from *Flavobacterium* sp. AJ 11199 (FERM-P4229) (Japanese Patent Application No. S56-025119 Publication). *Flavobacterium* sp. AJ 11199 (FERM-P4229) was initially deposited as *Alcaligenes aquamarinus* with the National Institute of Bioscience and Human-Technology, the Agency of Industrial Science of Technology, the Ministry of International Trade and Industry, JP, but as a result of re-identification, it was revealed that the microorganism should have been classified into *Flavobacterium* sp. *Flavobacterium* sp. AJ 11199 is a microorganism which was deposited on September 29, 1977, as FERM-P4229 with the National Institute of Bioscience and Human-Technology, the Agency of Industrial Science of Technology, the Ministry of International Trade and Industry, JP, and transferred on May 30, 2002 as FERM BP-8063 under the Budapest Treaty to International Patent Organism Depositary (IPOD), National Institute of Advanced Industrial Science and Technology (AIST).

The recombinant DNA carrying the genes encoding HHase, CHase and HRase is preferably a vector for introducing these genes into host cells. Examples of such vectors may include pHSG series (Takara Shuzo), pUC series (Takara Shuzo), pPROK series (Clontech), pSTV series (Takara Shuzo), pTWV series (Takara Shuzo), pKK233-2 (Clontech), a pBR322 series plasmid, and derivatives thereof. The "derivatives" are those obtained by modifying the plasmid by replacement, deletion, insertion, addition or inversion of a nucleotide. As referred to herein, the modification may include mutation treatment with a mutagen or UV irradiation, and modification by natural mutation. Depending on the type of the origin of replication, the copy number of the plasmids and derivatives thereof may vary in host cells. Examples of the high-copy plasmids may include pHSG series, pUC series and pPROK series plasmids, whereas examples of the low-copy plasmids may include pSTV series, pTWV series, pKK233 series and pBR322 series plasmids.

For increasing the amount of the protein production, it is preferable to connect a terminator, i.e. a transcription termination sequence, to a downstream region of the protein gene. Examples of the terminators may include rmB terminator, T7 terminator, fd phage terminator, T4 terminator, a terminator for tetracycline resistance gene, a terminator for *Escherichia coli* trpA gene, etc. A terminator such as rmB terminator is preferable in terms of improving the stability of the plasmid as well.

For facilitating selection of the transformant, the vector may preferably have a marker such as an ampicillin resistance gene and a kanamycin resistance gene.

A DNA fragment having the promoter, the DNA encoding HHase, the DNA encoding CHase, and the terminator each disposed therein at a predetermined location may be ligated to a vector DNA to give a recombinant DNA.

The recombinant DNA obtained in the aforementioned manner may be introduced into a host cell to give a transformant. In the production of the protein on a large scale with the recombinant DNA technology, the host cells to be transformed may be microbial cells, *Actinomyces* cells, yeast cells, fungal cells, plant cells, animal cells, etc. Because there are a lot of findings on production of proteins on a large scale by use of enterobacterium, it is generally preferable to use enterobacterium. In particular, *Escherichia coli* JM109, particularly (DE3) strain is preferable.

As another example of the process for producing an amino acid according to the present invention, HRase may be used together with HHase and CHase, as will be described in detail. In this case, HRase may be produced by the transformant as well, and then utilized in the production of the amino acid. That is, a recombinant DNA having a DNA encoding HRase may be prepared and then introduced into a host cell for transformation.

The DNA encoding HRase for use in the present invention may be those known in the art. Preferable examples thereof may be as follows:
(ix) a DNA having the base sequence of SEQ ID NO:5 in the Sequence Listing,
(x) a DNA having a base sequence that hybridizes under stringent conditions with a DNA having a base sequence complementary to the base sequence of SEQ ID NO:5 in the Sequence Listing,
(xi) a DNA encoding an amino acid sequence of SEQ ID NO:6 in the Sequence Listing, and
(xii) a DNA encoding an amino acid sequence wherein in the amino acid sequence of SEQ ID NO:6 in the Sequence Listing, one or several amino acid residues are replaced, deleted, inserted, added or inverted.
The terms "stringent conditions" and "several" herein have the same meaning as defined in the explanation of DNAs encoding HHase, etc.

The DNA of SEQ ID NO:5 in the Sequence Listing was isolated and purified from *Microbacterium liquefaciens* AJ 3912. *Microbacterium liquefaciens* AJ 3912 was initially deposited as *Flavobacterium* sp. AJ 3912 (FERM-P3133) on June 27, 1975 with the National Institute of Bioscience and Human-Technology, the Agency of Industrial Science of Technology, the Ministry of International Trade and Industry, Japan, but it was revealed as a result of re-identification that this microorganism should have been classified into *Aureobacterium liquefaciens*. Due to change of the name of this species, *Aureobacterium liquefaciens* has been re-classified into *Microbacterium liquefaciens*, and the microorganism has been deposited as *Microbacterium liquefaciens* AJ 3912 (National Deposition No. FERM-P3133 and International Deposition No. FERM BP-7643) with International Patent Organism Depositary (IPOD), National Institute of Advanced Industrial Science and Technology (AIST), Japan.

In the recombinant DNA carrying the DNA encoding HRase, it is preferable that the expression of the DNA encoding HRase is also regulated by the trc promoter. The terminator, the basic recombinant DNA, the selection marker and the host for the recombinant DNA having HRase gene integrated therein may preferably be the same as those described in the recombinant DNA carrying HHase gene, etc.

The recombinant DNA having the DNA encoding HRase may be introduced for expression into a host cell into which the DNA encoding HHase and the DNA encoding CHase have also been introduced, whereby the three enzymes for producing amino acids may be expressed by using one transformant, which makes the operation easier than in production using a plurality of transformants. In this case, the recombinant DNA having the DNA encoding HRase is preferably prepared separately from the recombinant DNA encoding HHase, etc. In consideration of the size of the enzyme gene and other factors, construction of a recombinant DNA by integrating the three enzyme genes into one recombinant DNA may not be practical. Accordingly, a transformant into which two sorts of recombinant DNAs were introduced is preferably prepared in order to express HRase as well.

Generally, simultaneous expression of two or more sorts of high-copy recombinant DNAs in one host cell is difficult. It is thus preferable that, when two or more sorts of recombinant DNAs are to be introduced, one of the recombinant DNAs is a high-copy recombinant DNA, and the other is a relatively low-copy recombinant DNA. The amount of the enzyme produced by each DNA, the stability of the plasmid, etc. may be taken into consideration for determining which recombinant DNA are incorporated into the high-copy recombinant DNA. In the production of the amino acid with the recombinant DNA of the present invention, it is preferable to incorporate the recombinant DNA having HRase into a high-copy recombinant DNA, and the recombinant DNA having HHase and CHase into a low-copy recombinant DNA. Whether the DNA is high-copy or not may be judged by preparing transformants having each of the recombinant DNAs and measuring the activity of the enzyme produced by each transformant.

Procedures for handling plasmids, DNA fragments, various enzymes, preparation of transformants, and screening of the transformants in the present invention may be carried out in accordance with known techniques described in Molecular Cloning, A Laboratory Manual, 2nd Edition, edited by J. Sambrook et al., 1989, Cold Spring Harbor Laboratory Press, etc.

### 2. Process for producing the enzyme protein

HHase and CHase may be expressed and produced in a large amount by culturing cells that have been transformed with the recombinant DNA as described above. When the DNA encoding HRase has also been introduced, HRase may also be expressed and produced. As the medium for the production, media that are usually used in the cultivation of *E*. *coli*, for example M9-casamino acid medium, LB medium, etc. may be used. Specific conditions for carrying out the cultivation and inducing the production may suitably be selected depending on the types of the marker in the employed vector, the host microorganism, etc.

After the cultured transformant cells are collected by centrifugation, etc., the transformant cells may be disrupted or lyzed. HHase and/or CHase may then be recovered therefrom, which may be used as a crude enzyme solution. The disruption may by performed by the methods such as ultrasonic disruption, French press disruption, glass bead disruption, etc. Lysis of the microorganism may be performed by treatment with egg white lysozyme or a peptidase, or any suitable combination thereof. If necessary, these enzymes may be purified before use. The purification may be performed by conventional techniques such as precipitation, filtration and column chromatography. A purification method with antibodies to the enzymes may also be used.

In a preferable example for the production of the protein on a large scale by recombinant DNA technology, the protein may be aggregated in the transformant producing the protein to constitute a protein inclusion body. The advantage of this production method lies in protection of the desired protein against digestion with proteases present in the microorganism as well as in easy purification of the desired protein by disruption and centrifugation of the microorganism.

The protein inclusion body thus obtained may be solubilized by denaturation of the protein. After the restoration of the activity by e.g., removing the denaturant, the inclusion body may be converted into a correctly folded, physiologically active protein. There are many examples of such conversion, e.g. restoration of the activity of human interleukin-2 (Japanese Patent Application No. S61-257931 Publication).

Retrieval of the active protein from the protein inclusion body may require a series of procedures such as solubilization and activity restoration, etc., which may make the procedures more complex than in direct production of the active protein. However, if the protein to be produced in the cells in a large amount may affect growth of the microorganism, accumulation of the protein in the form of such an inert inclusion body in the cells may contribute to suppress influence of such a protein on the microorganism.

Production of the objective protein as the inclusion body on a large scale may be performed by sole expression of the desired protein under the control of a strong promoter, as well as expression of a fused protein consisting of the objective protein and another protein that is known to be expressed in a large amount.

It may be useful to dispose a sequence that is recognizable by a restriction protease in a suitable position for cutting the objective protein out after the expression of the fused protein.

The protein inclusion body thus formed as the fused protein may be recovered and then solubilized with a denaturant The inclusion body may be solubilized together with microbial proteins. However, considering the subsequent purification procedures, it is preferable to take the inclusion body out before solubilization. The inclusion body may be recovered from the microorganism by a method known in the art. For example, the microorganism may be disrupted and the inclusion body may then be recovered by treatments such as centrifugation. Examples of the denaturant for solubilizing the protein inclusion body may include guanidine hydrochloride (e.g., 6 M, pH 5 to 8) and urea (e.g. 8 M).

The enzyme protein may be regenerated as an active protein by dialysis for removing the denaturant The dialysis solution used in dialysis may be Tris-HCl buffer or phosphate buffer, and the concentration thereof may be 20 mM to 0.5 M, and the pH thereof may be 5 to 8.

The concentration of the protein in the regeneration step is preferably regulated to be no grater than about 500 µg/ml. In order to prevent self-crosslinkage of the regenerated enzyme protein, the dialysis temperature is preferably 5°C or less. In addition to this dialysis method, examples of the method for removing the denaturant may further include a dilution method and an ultrafiltration method, with any of which the activity may be restored.

### 3. Process for producing an amino acid

In the process for producing the amino acid according to the present invention, the amino acid may be produced by culturing in a medium a transformed cell that has been obtained by introducing the recombinant DNA encoding enzymes such as HHase as described above to give a cultured product containing the enzymes such as HHase formed therein, and then mixing the cultured product with the 5-substituted hydantoin to form an amino acid. The cultured product may contain HHase and CHase if the DNA encoding HHase and CHase has been introduced into the transformed cell. The resulting cultured product may also contain HRase if the DNA encoding HRase has also been introduced. Insofar as these predetermined enzymes are contained, the form of the cultured product is not particularly limited. That is, the 5-substituted hydantoin may be added directly to a medium containing the transformant, or the 5-substituted hydantoin may be mixed with the transformant such as the microorganism separated from a medium, a washed transformant, a treated material obtained by disrupting or lyzing the transformant, a crude enzyme solution containing recovered HHase, etc., or a purified enzyme solution, whereby the desired amino acid can be produced.

The reaction steps for forming an amino acid from a 5-substitued hydantoin with HHase and CHase is shown in the following reaction scheme (I):

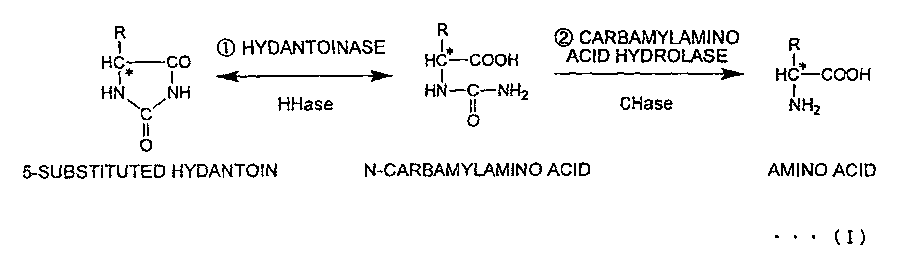

If it is desired to produce an optically active amino acid, HHase having optical selectivity may be used to form either N-carbamyl-L-amino acid or N-carbamyl-D-amino acid. The carbamylamino acid may then be subjected to the reaction with CHase, to produce the optically active amino acid.

Even if HHase has no optical selectivity in hydrolysis activity, employment of CHase having an optical selectivity will result in the production of the optically active D-or L-amino acid. In this case, it may be predicted that the unreacted one of the N-carbamylamino acid enantiomers would remain in the reaction liquid; that is, if CHase selectively decomposes N-carbamyl-L-amino acid to produce the L-amino acid, N-carbamyl-D-amino acid would remain in the reaction liquid, while if CHase selectively produces D-amino acid, N-carbamyl-L-amino acid would remain. In this case, however, HHase can also slightly catalyze the reverse reaction of dehydrating and condensing the remaining unreacted enantiomer of N-carbamylamino acid to form the 5-substituted hydantoin compound again, and therefore the optically active amino acid may be produced with high yield (at least 50 percent molar yield) by the two enzymes, that is, HHase and highly optically selective CHase or by a material containing such two enzymes.

Subsequently, as an example of the reaction for forming an optically active amino acid from DL-5-substituted hydantoin, the reaction for optically selective production of D-amino acid by HRase is shown in the reaction scheme (II):

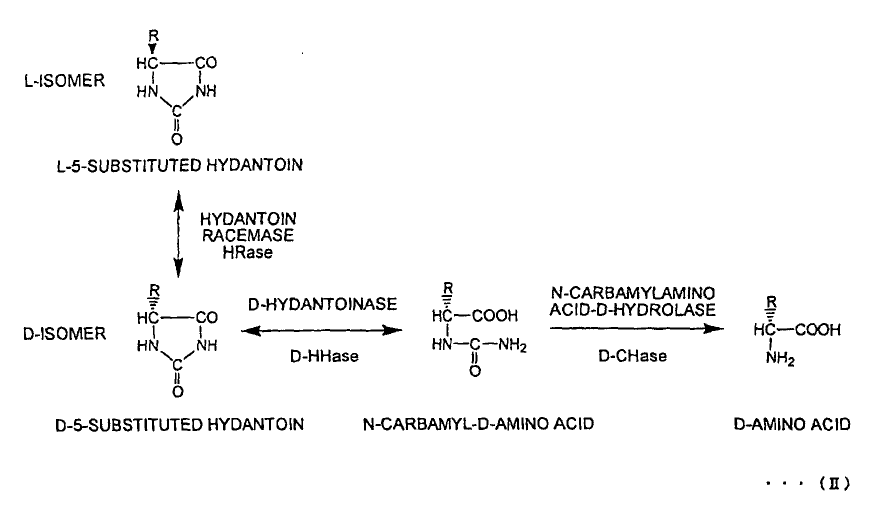

Since HRase in the protein mixture catalyzes the racemization of the 5-substituted hydantoin compound as shown in the reaction scheme (II), employment of HRase theoretically enables production of the D-amino acid with a molar yield of 100 percent from the DL-5-substituted hydantoin compound.

The process for producing the amino acid of the present invention employs the 5-substituted hydantoin compounds as the substrate for HHase or HRase. Examples of the 5-substituted hydantoin compounds may include 5-substituted hydantoin compounds corresponding to the natural amino acids such as hydantoin, 5-methyl hydantoin, 5-benzyl hydantoin, 5-(4-hydroxybenzyl)hydantoin, 5-indolylmethyl hydantoin, 5-(3,4-dihydroxybenzyl)hydantoin, 5-(p-hydroxybenzyl)hydantoin, 5-(3'-pyridyl)-methyl hydantoin, 5-methyl thioethyl hydantoin, 5-isopropyl hydantoin, 5-isobutyl hydantoin, 5-sec-butyl hydantoin, 5-carboxyethyl hydantoin, 5-carboxymethyl hydantoin, 5-(4-aminobutyl)hydantoin, and 5-hydroxymethyl hydantoin, as well as 5-substituted hydantoins corresponding to artificial amino acids such as 5-phenyl hydantoin, 5-(4-hydroxyphenyl)hydantoin, 5-methoxymethyl hydantoin, 5-benzyloxymethyl hydantoin, and 5-(3,4-methylenedioxybenzyl)hydantoin, and dihydrouracil, or derivatives thereof.

The enzyme proteins may also be used to produce N-carbamylamino acid. For example, it is possible to produce an N-carbamylamino acid by adding an L- or D-CHase inhibitor to the mixture of proteins to terminate the hydrolysis reaction at the stage of N-carbamylamino acid.

When a cultured liquid of cells, separated cells, washed cells, or treated cells that have been transformed with the recombinant DNA, or a crude enzyme solution or a purified enzyme obtained from the treated microbial material are used for catalyzing the amino acid-forming reaction, a reaction liquid containing the 5-substituted hydantoin compound and the cultured liquid, the separated cells, the washed cells, the treated cells, the crude enzyme solution, or the purified enzyme may be left stand or stirred for 8 hours to 5 days in a suitable temperature in a range of 25 to 40°C while the pH is kept at 5 to 9.

When a water-soluble medium is employed for the cultivation of the cells that have been transformed with the recombinant DNA and the simultaneous amino acid-forming reaction, the water-soluble medium therefor may contain the 5-substituted hydantoin compound and nutrients such as a carbon source, a nitrogen source, and inorganic ions necessary for growth of the transformed cells. Further, addition of organic micronutrients such as vitamins, amino acids, etc. leads to desirable results in most of cases. An amount of the 5-substituted hydantoin compound to be added may be divided in portions and added separately. Culturing may preferably be conducted for 8 hours to 5 days under aerobic conditions while maintaining suitable pH and temperature in ranges of pH 5 to 9 and 25 to 40°C, respectively.

The amount of the D-amino acid in the cultured liquid or reaction liquid may be rapidly determined by a method known in the art. Simple measurement may be thin layer chromatography with, e.g. HPTLC CHIR manufactured by Merck. Measurement with higher analytical accuracy may be performed by high performance liquid chromatography (HPLC) with an optical resolution column such as CHIRALPAK WH manufactured by Daicel Chemical Industries, Ltd.

The amino acid thus produced may be separated and purified by techniques known in the art Examples of the method therefor may include adsorption of basic amino acid by contacting the same with an ion-exchange resin which is followed by elution and crystallization thereof. Alternatively, the eluent may be passed through activated charcoal for discoloration and then crystallized.

Although it depends on the conditions for culturing the transformant such as *Escherichia coli*, employment of the recombinant DNA of the present invention using the trp promoter may achieve productivity (amount of amino acids per unit time) of approximately five times as the productivity achieved with other promoters such as rhamnose promoter.

### Examples

The present invention is further described in detail with reference to Examples 1 to 8. However, the present invention is not limited thereto.

### (Example 1) Expression of D-hydantoinase gene derived from AJ 11199 strain in E. coli

### 1. Construction of an expression plasmid

### 1-1. Construction of a plasmid carrying a trp promoter

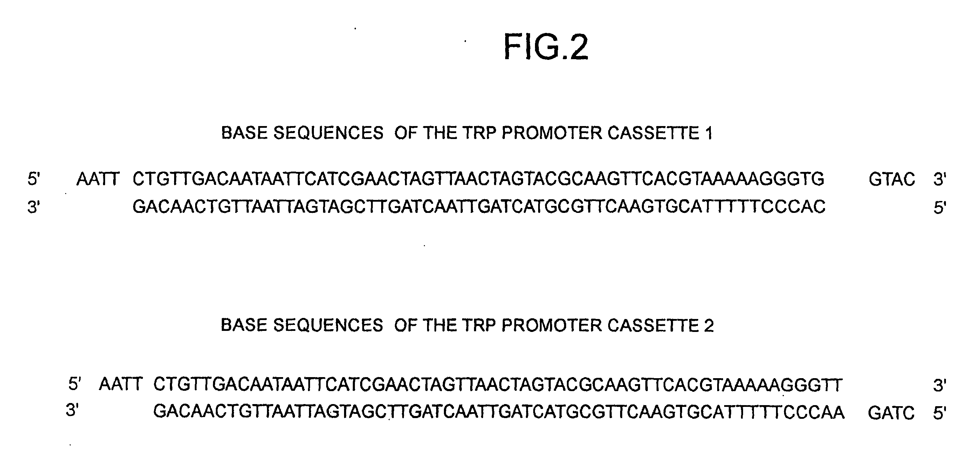

pHSG298 (manufactured by Takara Shuzo Co., Ltd.) was treated with EcoRI/KpnI, and the resulting DNA fragment was ligated to a trp promoter cassette 1 (Fig. 2). The solution of this ligation product was used to transform *E. coli* JM109. Among the resulting kanamycin-resistant strains, a strain having the objective plasmid was selected, and this plasmid was designated pTrp298EK. In the Sequence Listing, SEQ ID NO:21 describes the upper chain of the trp promoter cassette 1 shown in Fig. 2.

### 1-2. Construction of a plasmid carrying a D-hydantoinase gene

The objective gene was amplified by PCR with a chromosomal DNAfrom *Flavobacterium* sp. AJ 11199 as a template and the oligonucleotides shown in Table 1 as primers (four combinations, wherein one of (1) to (4) was combined with (5)). These fragments were treated with KpnI/XbaI, and the resulting DNA fragments were ligated to pTrp298EK that had previously been treated with KpnI/XbaI. The solution containing this ligation product was used to transform *E*. *coli* JM109. Among the resulting kanamycin-resistant strains, strains having the objective plasmids were selected. The plasmids carrying the DNA fragment that had been amplified with one of the primers (1) to (4) and the primer (5) were designated pTrp298DHHase1, 2, 3, and 4, respectively.

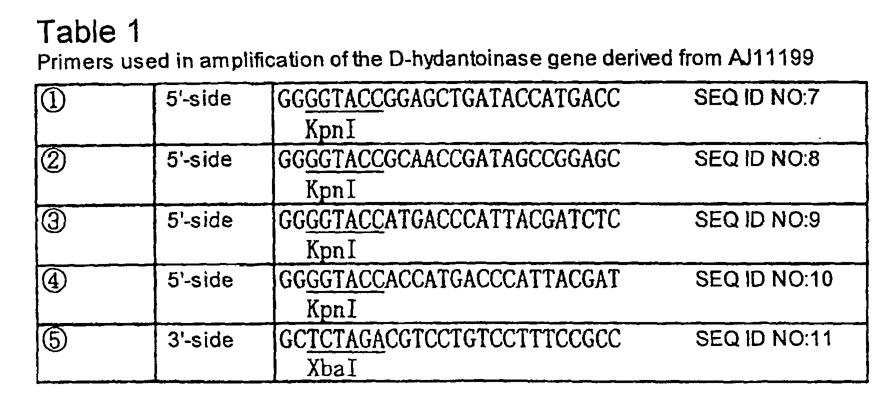

### 2. Preparation of a cell-free extract

*E*. *coli* JM109 having pTrp298DHHase1, 2, 3, or 4 was cultured at 30°C for 16 hours in LB medium (2 ml) containing 100 µg/ml kanamycin. 100 µl of the cultured liquid thus obtained was transferred to 4 ml of medium I (1 percent casamino acid, 1 percent peptone, 1 percent glucose, 0.5 percent ammonium sulfate, 0.1 percent monopotassium phosphate, 0.3 percent dipotassium phosphate, 0.05 percent magnesium sulfate, 0.01 percent kanamycin (pH 7.0)), and cultured at 37°C for 16 hours. After the cultivation, the microorganism was collected from 1 ml of the cultured liquid by centrifugation (10,000 g×10 minutes), and the resulting microorganism was washed with 100 mM potassium phosphate buffer (KPB, pH 7.5). This washed microorganism was suspended in 0.5 ml of the same buffer and disrupted by ultrasonic disruption (20 kHz, 15 minutes, with UCW-201 manufactured by Tohsho Denki). The liquid obtained by the ultrasonic disruption was centrifuged (10,000 g×10 minutes) to give a supernatant as a cell-free extract.

### 3. Confirmation of D-hydantoinase gene expression by SDS polyacrylamide gel electrophoresis

10 µl of the cell-free extract was applied to an SDS polyacrylamide gel (Multigel 10/20 manufactured by Daiichi Pure Chemicals Co., Ltd.) and electrophoresed in accordance with the method of Laemelli et al. As a result, a band of a protein corresponding to D-hydantoinase was recognized only in the cell-free extract of the transformant having pTrp298DHHase3.

### 4. Measurement of D-hydantoinase activity

The *E. coli* JM109 having pTrp298DHHase3 was cultured at 30°C for 16 hours in LB medium (2 ml) containing 100 µg/ml kanamycin. 0.5 ml of the cultured liquid thus obtained was transferred to 50 ml of medium II (1 percent casamino acid, 1 percent peptone, 0.2 percent glucose, 0.5 percent ammonium sulfate, 0.1 percent monopotassium phosphate, 0.3 percent dipotassium phosphate, 0.05 percent magnesium sulfate, 0.01 percent manganese sulfate, 0.01 percent kanamycin (pH 7.0)) and cultured at 37°C for 24 hours.

After the cultivation, the microorganism was collected from 5 ml of the cultured liquid by centrifugation (10,000 g×10 minutes) and washed with 100 mM KPB (pH 7.5). The microorganism thus washed was suspended in 5 ml of the same buffer, and 0.1 ml of this suspension was added to 0.9 ml of a substrate solution (133 mg/dl D-5-benzylhydantoin, 1.1 mM MnSO₄, 100 mM KPB (pH 7.5)) and reacted at 37°C for 60 minutes. After the reaction, 0.9 ml of a solution for terminating the reaction (1.1 mM CuSO₄) was added to 0.1 ml of the reaction liquid and then centrifuged, and the resulting supernatant was subjected to HPLC (column: CHIRALPAK WH, 0.46×25 cm, eluting solution: 1 mM CuSO₄, flow rate: 1.5 ml/min., temperature: 50°C, detection wavelength: 210 nm) to quantify the formed N-carbamyl-D-Phe. One unit of the enzyme was defined as the amount of the enzyme that produces 1 µmol of N-carbamyl-D-Phe per minute under these conditions.

As a result, the cultured liquid of the *E*. *coli* JM109 having pTrp298DHHase3 exhibited1.5 units per mL of D-hydantoinase activity.

### (Example 2) Expression of D-carbamylase gene derived from AJ 11199 strain in E. coli

### 1. Construction of a plasmid carrying a D-carbamylase gene

The objective gene was amplified by PCR with a chromosomal DNAfrom *Flavobacterium* sp. AJ 11199 as a template and the oligonucleotides shown in Table 2 as primers (two combinations wherein either (1) or (2) was combined with (3)). These fragments were treated with KpnI/XbaI, and the resulting DNAfragments were ligated to pTrp298EK that had previously been treated with KpnI/XbaI. The solution containing this ligation product was used to transform *E*. *coli* JM109. Among the resulting kanamycin-resistant strains, strains having the objective plasmids were selected. The plasmids carrying a fragment that had been amplified with one of the primers (1) or (2) and the primer (3) were designated pTrp298DCHase1 and 2, respectively.

Then, pTrp298DCHase1 was treated with EcoRI/XbaI to give a DNAfragment having the trp promoter and the carbamylase gene ligated thereto, and this DNA fragment was ligated to pHSG299 (Takara Shuzo) that had previously been treated with EcoRI/XbaI. The solution containing this ligation product was used to transform E. coli JM109. Among the resulting kanamycin-resistant strains, a strain having the objective plasmid was selected, and the plasmid therein was designated pTrp299DCHase1.

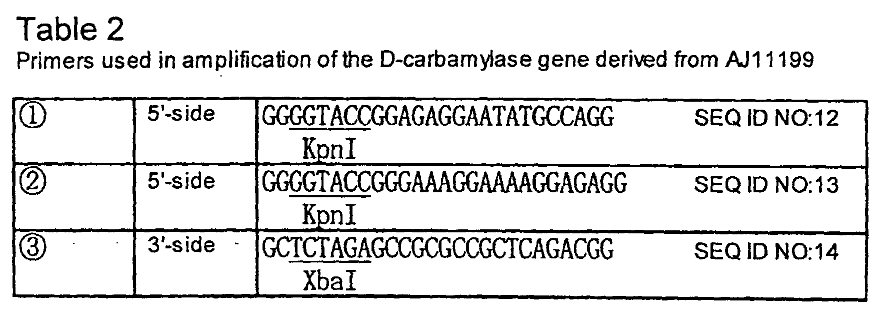

### 2. Preparation of a cell-free extract

Preparation of a cell-free extract of the *E*. *coli* JM109 having pTrp298DCHase1, 2, or pTrp299DCHase1 was conducted in the same manner as in the preparation of the cell-free extract described in Example 1.

### 3. Confirmation of D-carbamylase gene expression by SDS polyacrylamide gel electrophoresis

10 µl of the cell-free extract was applied to an SDS polyacrylamide gel (Multigel 10/20 manufactured by Daiichi Pure Chemicals Co., Ltd.) and electrophoresed in accordance with the method of Laemelli et al. As a result, a band of a protein corresponding to D-carbamylase was recognized only in the cell-free extract of the transformant having pTrp299DCHase1.

### 4. Measurement of D-carbamylase activity

Preparation of cells of the *E*. *coli* JM109 having pTrp299DCHase1 was conducted in the same manner as the preparation performed in the measurement of enzyme activity described in Example 1. The washed microorganism obtained thereby was suspended in 5 ml of 100 mM KPB (pH 7.5), and 0.1 ml of this suspension was added to 0.9 ml of a substrate solution (458 mg/dl N-carbamyl-D-Phe, 100 mM KPB (pH 7.5)) and reacted at 37°C for 60 minutes. After the reaction, 0.9 ml of the solution for terminating the reaction was added to 0.1 ml of the reaction liquid and then centrifuged, and the resulting supernatant was subjected to HPLC to quantify the formed D-Phe. One unit of the enzyme was defined as the amount of the enzyme that produces 1 µmol of D-Phe per minute under these conditions.

As a result, the cultured liquid of the *E*. *coli* JM109 having pTrp299DCHase1 exhibited 0.6 unit per mL of D-carbamylase activity.

### (Example 3) Expression of hydantoin racemase gene derived from AJ 3912 in E. coli

### 1. Construction of an expression plasmid

### 1-1. Construction of a plasmid carrying a trp promoter

pHSG298 and pHSG299 were treated with EcoRI/XbaI, and each of the resulting DNA fragments was ligated to a trp promoter cassette 2 (Fig. 2). The solution of each of these ligation products was used to transform *E*. *coli* JM109. Among the resulting kanamycin-resistant strains, strains having the objective plasmids were selected, and these plasmids were designated pTrp298EK and pTrp299EX, respectively. In the Sequence Listing, SEQ ID NO:22 describes the upper chain of the trp promoter cassette 2 shown in Fig. 2.

### 1-2. Construction of a plasmid carrying a hydantoin racemase gene

The objective gene was amplified by PCR with a chromosomal DNA from *Microbacterium liquefaciens* AJ 3912 as a template and the oligonucleotides shown in Table 3 as primers (two combinations wherein either (1) or (2) was combined with (3)). These fragments were treated with XbaI/PstI, and each of the resulting DNAfragments was ligated to each of pTrp298EK and pTrp299EX that had previously been treated with XbaI/PstI. Each solution containing each of these ligation products was used to transform *E*. *coli* JM109, and from the resulting kanamycin-resistant strains, strains having the objective plasmids were selected. The plasmids carrying a DNA fragment that had been amplified with one of the primers (1) or (2) and the primer (3) were designated pTrp298HRase1, 2, and pTrp299HRase 1, 2, respectively.

Then, pTrp299HRase2 was treated with EcoRI/PstI to give a DNA fragment having the trp promoter and the hydantoin racemase gene ligated thereto, and this DNA fragment was ligated to pSTV29 (Takara Shuzo) that had previously been treated with EcoRI/PstI. The solution containing this ligation product was used to transform *E*. *coli* JM109. Among the resulting chloramphenicol-resistant strains, a strain having the objective plasmid was selected, and the plasmid was designated pTrp29HRase.

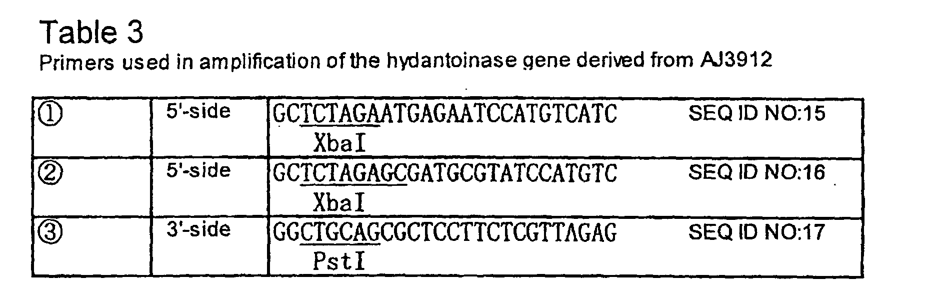

### 2. Measurement of hydantoin racemase activity

Preparation of the *E*. *coli* JM109 having pTrp298HRase1 or 2, pTrp299HRase1 or 2, or pTrp29HRase was conducted in the same manner as the preparation performed in the measurement of enzyme activity described in Example 1. Each of the washed microorganisms obtained thereby was suspended in 5 ml of 100 mM KPB (pH 7.5), and 0.1 ml of this suspension was added to 0.9 ml of a substrate solution (133 mg/dl D-5-benzylhydantoin, 100 mM KPB (pH 7.5)) and reacted at 37°C for 15 minutes. After the reaction, 0.9 ml of the solution for terminating reaction was added to 0.1 ml of the reaction liquid and then centrifuged, and the resulting supernatant was subjected to HPLC to quantify the formed L-5-benzylhydantoin. One unit of the enzyme was defined as the amount of the enzyme that produces 1 µmol of L-5-benzylhydantoin per minute under these conditions. The results are shown in Table 4.

**Table 4**

| Hydantoinase racemase activity of the transformant having each plasmid | |
|---|---|
| Plasmid | Hydantoin racemase activity (U/ml culture liquid) |
| pTrp298HRase-1 | 4.47 |
| pTrp298HRase-2 | 0.31 |
| pTrp299HRase-1 | 6.24 |
| pTrp299HRase-2 | 7.76 |
| pTrp29HRase | 0.45 |

### (Example 4) Co-expression of D-hydantoinase gene and D-carbamylase gene derived from AJ 11999 in E. coli

### 1. Construction of a co-expression plasmid

### 1-1. Construction of pTrpHfCf plasmid

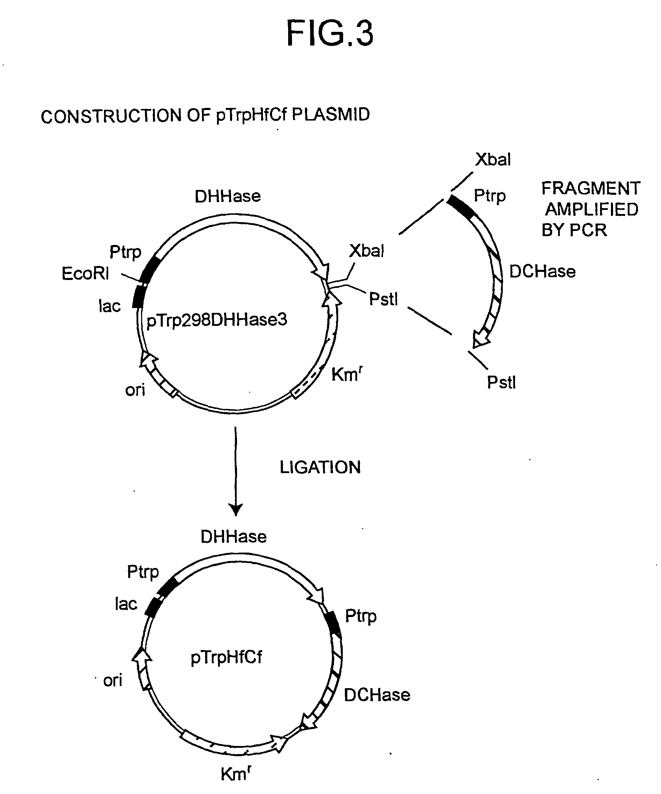

PCR was conducted with pTrp299DCHase1 as a template and the oligonucleotides (1) and (2) shown in Table 5 as primers. The resulting DNA fragment was treated with XbaI/PstI and then ligated to pTrp298DHHase3 that had previously been treated with XbaI/Pst. The solution of this ligation product was used to transform *E*. *coli* JM109. Among the resulting kanamycin-resistant strains, a strain having the objective plasmid was selected, and this plasmid was designated pTrpHfCf (Fig. 3).

### 1-2. Construction of pTrpCrHf plasmid

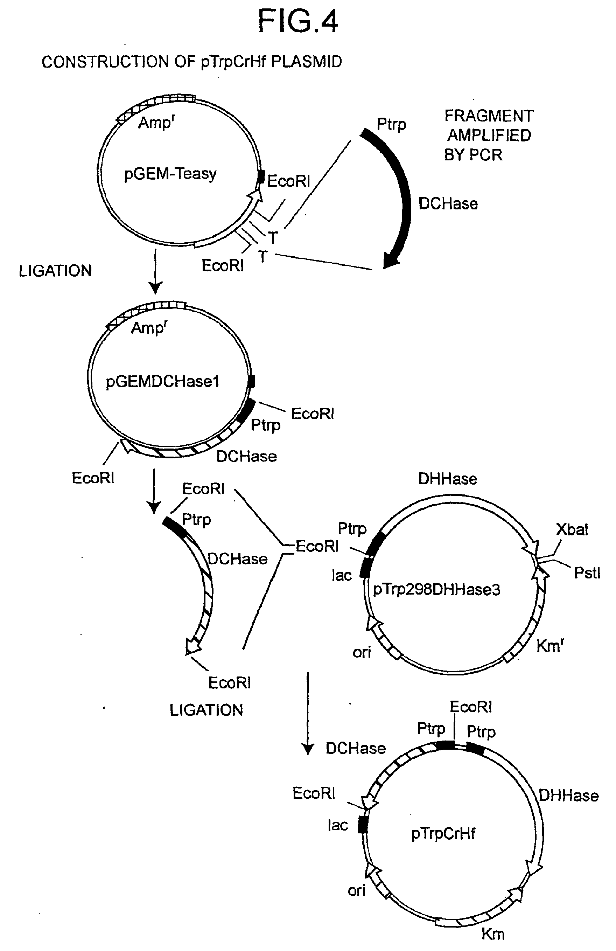

PCR was conducted with pTrp299DCHase1 as a template and the oligonucleotides (1) and (2) shown in Table 5 as primers. The resulting DNA fragment was ligated to pGEM-Teasy vector (Promega). The solution of the ligation product was used to transform *E*. *coli* JM109. Among the resulting ampicillin-resistant strains, a strain having the objective plasmid was selected, and this plasmid was designated pGEMDCHase1. Then, this plasmid was treated with EcoRI to give a DNA fragment having the trp promoter and the carbamylase gene ligated thereto, and this DNA fragment was ligated to pTrp298DHHase3 that had previously been treated with EcoRI. The solution containing the ligation product was used to transform *E*. *coli* JM109. Among the resulting kanamycin-resistant strains, a strain having the objective plasmid was selected, and the plasmid was designated pTrpCrHf (Fig. 4).

### 1-3. Construction of pTrpHrCr plasmid

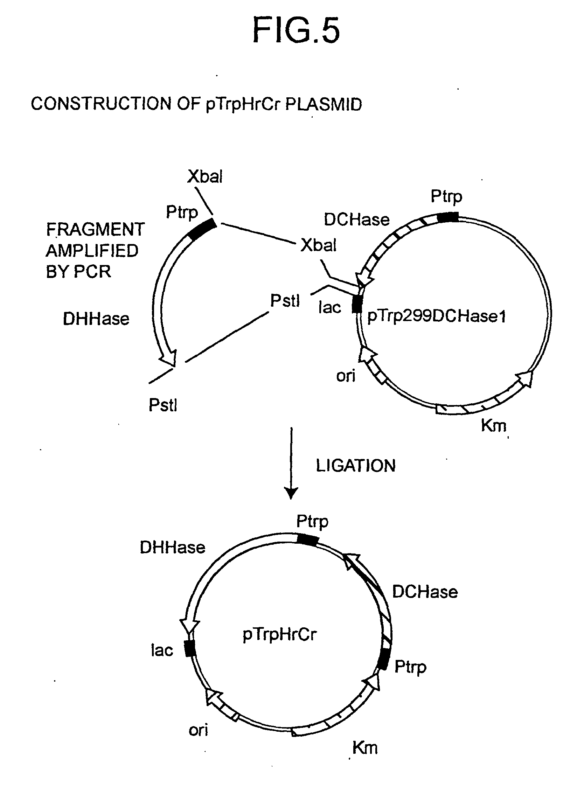

PCR was conducted with pTrp298DHHase3 as a template and the oligonucleotides (1) and (3) shown in Table 5 as primers. The resulting DNAfragment was treated with XbaI/PstI and then ligated to pTrp299DCHase1 that had previously been treated with XbaI/Pst. The solution containing the ligation product was used to transform *E*. *coli* JM109. Among the resulting kanamycin-resistant strains, a strain having the objective plasmid was selected, and this plasmid was designated pTrpHrCr (Fig. 5).

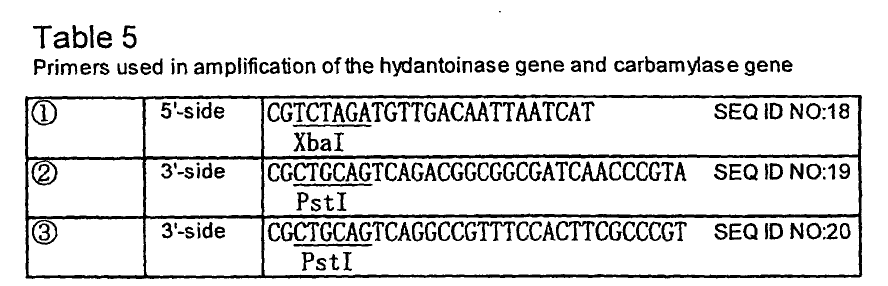

### 2. Measurement of enzyme activity

Preparation of the microorganism *E*. *coli* JM109 having plasmid pTrpHfCf, pTrpCrHf, or pTrpHrCr was conducted in the same manner as the preparation performed in the measurement of enzyme activity described in Example 1. 0.2 ml of the resulting microbial suspension was added to 0.8 ml of a substrate solution (0.125 g/dl DL-benzylhydantoin, 1.25 mM MnSO₄, 100 mM KPB (pH 7.5)) and reacted at 37°C for 60 minutes. After the reaction, 0.9 ml of the solution for terminating the reaction was added to 0.1 ml of the reaction liquid. A supernatant obtained by centrifugation was subjected to HPLC, and the amounts of formed N-carbamyl-D-Phe and D-Phe were determined. The results are shown in Table 6. In the transformant having any one of the plasmids, the co-expression of D-hydantoinase gene and D-carbamylase gene was recognized.

**Table 6**

| Enzyme activity of the microorganism co-expressing D-hydantoinase gene and D-carbamylase gene | | |
|---|---|---|
| Plasmid | D-hydantoinase activity (U/ml culture liquid) | D-carbamylase activity (U/ml culture liquid) |
| pTrpHfCf | 0.09 | 0.02 |
| pTrpCrHf | 0.43 | 0.17 |
| pTrpHrCr | 0.90 | 0.66 |

### (Example 5) Co-expression of D-hydantoinase gene, D-carbamylase gene and hydantoin racemase gene in E. coli

pTrp29HRase was introduced into the *E*. *coli* JM109 having pTrpHrCr to prepare a transformant having the two plasmids. This transformant was cultured at 30°C for 16 hours in LB medium (4 ml) containing 100 µg/ml kanamycin and 50 µg/ml chloramphenicol. 1 ml of the cultured liquid thus obtained was transferred to 50 ml of medium II and cultured at 35°C for 24 hours. After the cultivation, the microorganism was collected from 1 ml of the cultured liquid by centrifugation (10,000 g×10 minutes), and the resulting microorganism was washed with 100 mM KPB (pH 7.5). The washed microorganism was suspended in 1 ml of the same buffer, and 0.1 ml of the microbial suspension was added to 0.9 ml of a substrate solution (0.11 g/dl L-5-benzylhydantoin, 1.1 mM MnSO₄, 0.11 percent Triton X-100, 100 mM KPB (pH 7.5)) and reacted at 37°C for 60 minutes. After the reaction, the amount of formed D-Phe was determined. It was found out that 92.7 mg/dl of D-Phe was formed, whereby co-expression of these three enzyme genes was confirmed.

### (Example 6) Production of D-Phe with the microorganism co-expressing D-hydantoinase gene, D-carbamylase gene and hydantoin racemase gene

The *E*. *coli* JM109 having two plasmids, i.e. pTrpHrCr and pTrp29HRase, was cultured at 30°C for 16 hours in LB medium (50 ml) containing 100 µg/ml kanamycin and 50 µg/ml chloramphenicol. 15 ml of the cultured liquid thus obtained was transferred to 300 ml of medium III (1 percent casamino acid, 1 percent peptone, 2 percent glucose, 0.5 percent ammonium sulfate, 0.1 percent monopotassium phosphate, 0.3 percent dipotassium phosphate, 0.05 percent magnesium sulfate, 0.01 percent manganese sulfate, 0.01 percent kanamycin, 0.005 percent chloramphenicol (pH 7.0)) and cultured at 35°C for 15 hours in a jar fermenter (adjusted to pH 7.0). 30 ml of the cultured liquid (300 mg as the dry weight of the microorganism) thus obtained was added to 270 ml of a substrate solution (5.5 g/dl DL-5-benzyl hydantoin, 1.11 mM MnSO₄, 0.11 percent Triton X-100, 22 mM KPB (pH 7.5)) and reacted at 37°C for 44 hours while the pH of the reaction liquid was kept at pH 7.5 (adjusted with 1 N NaOH and 2 N H₂SO₄). As a result, it was recognized that 4.13 g/dl (molar yield, 95 percent) of D-Phe was produced.

### (Example 7) Production of D-Phe with the microorganism co-expressing D-hydantoinase gene, D-carbamylase gene and hydantoin racemase gene (cultured in an antibiotic-free medium)

The *E*. *coli* JM109 having two plasmids, i.e. pTrpHrCr and pTrp29HRase, was cultured in kanamycin- and chloramphenicol-free LB medium (50 ml) at 30°C for 16 hours. 15 ml of the cultured liquid thus obtained was cultured at 35°C for 15 hours in 30 ml of kanamycin- and chloramphenicol-free medium III in a jar fermenter (adjusted to pH 7.0). 30 ml of the cultured liquid (300mg as the dry weight of the microorganism) thus obtained was added to 270 ml of a substrate solution (5.5 g/dl DL-5-benzyl hydantoin, 1.11 mM MnSO₄, 0.11 percent Triton X-100, 22 mM KPB (pH 7.5)) and reacted at 37°C for 44 hours while the pH of the reaction liquid was kept at pH 7.5 (adjusted with 1 N NaOH and 2 N H₂SO₄). As a result, it was recognized that 2.06 g/dl (molar yield, 48 percent) of D-Phe was produced.

### (Example 8)

### 1-1. Construction of a plasmid carrying trp promoter and rmB terminator

A trp operon promoter region in the chromosomal DNA of *Escherichia coli* W3110 was amplified as an objective gene region by PCR with the oligonucleotides shown in Table 7 as primers (combination of (1) and (2)). The resulting DNAfragment was ligated to pGEM-Teasy vector (Promega). The solution containing the ligation product was used to transform *E. coli* JM109. Among the resulting ampicillin-resistant strains, a strain having the objective plasmid in which the trp promoter was inserted in the reverse direction of the lac promoter was selected.

This plasmid was then treated with EcoO109I/EcoRI to obtain a DNA fragment containing the trp promoter, which was further ligated to pUC19 (Takara Shuzo) that had previously been treated with EcoO109I/EcoRI. The solution containing the ligation product was used to transform *E*. *coli* JM109. Among the resulting ampicillin-resistant strains, a strain having the objective plasmid was selected, and the plasmid was designated pTrp1.

pKK223-3 (Amersham Pharmacia) was then treated with HindIII/HincII, and the resulting DNA fragment containing rmB terminator was ligated to pTrp1 that had previously been treated with HindIII/PvuII. The solution containing the ligation product was used to transform *E. coli* JM109. Among the resulting ampicillin-resistant strains, a strain having the objective plasmid was selected, and the plasmid was designated pTrp2.

The trp promoter region was then amplified by PCR with pTrp2 as a template and the oligonucleotides in the table (combination of (1) and (3)) as primers. This DNA fragment was treated with EcoO109I/Ndel and then ligated to pTrp2 that had previously been treated with EcoO109I/Ndel. The solution containing the ligation product was used to transform *E*. *coli* JM109. Among the resulting ampicillin-resistant strains, a strain having the objective plasmid was selected, and the plasmid was designated pTrp4.

A DNA fragment of 2.4 kb obtained by treating pSTV28 (Takara Shuzo) with EcoO109I/Pvul, a DNA fragment of 0.9 kb obtained by treating pKK223-3 (Amersham Pharmacia) with HindIII/Pvul, and a DNAfragment of 0.3 kb obtained by treating pTrp4 with EcoO109I/HindIII were ligated. The solution containing the ligation product was used to transform *E. coli* JM109. Among the resulting chloramphenicol-resistant strains, a strain having the objective plasmid was selected, and the plasmid was designated pTrp8.

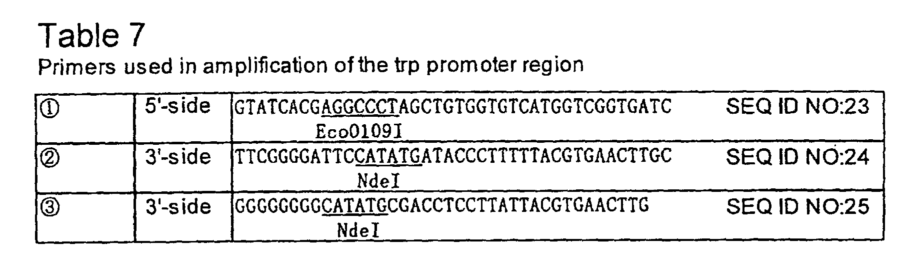

### 1-2. Construction of a plasmid carrying a hydantoin racemase gene

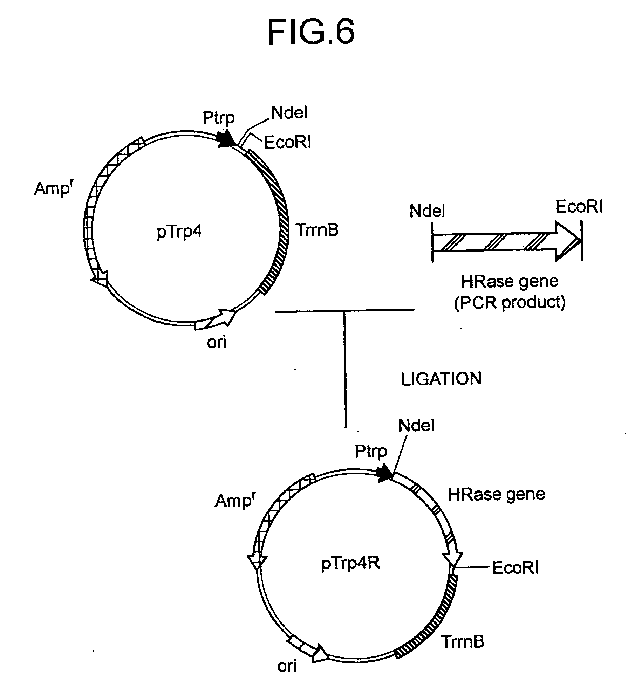

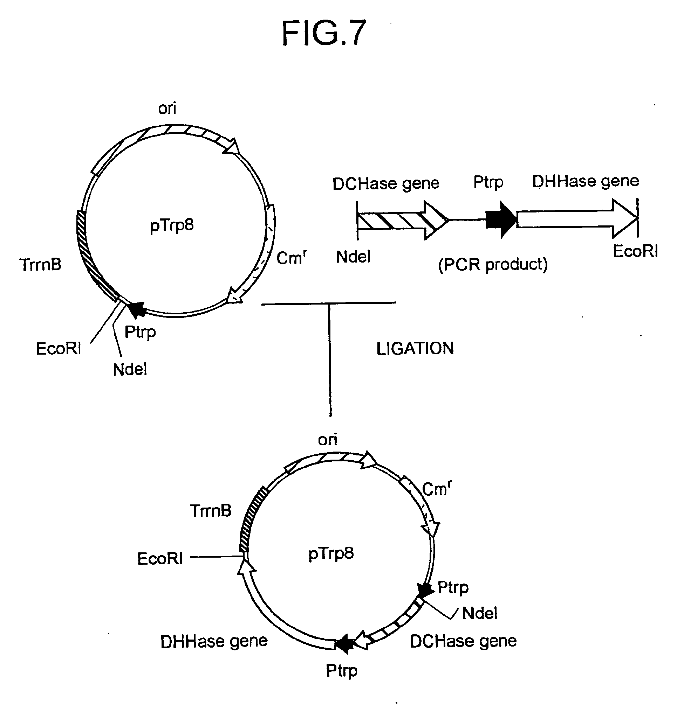

The objective gene was amplified by PCR with a chromosomal DNAfrom *Microbacterium liquefaciens* AJ 3912 as a template and the oligonucleotides shown in Table 8 as primers. This fragment was treated with NdeI/EcoRI, and the resulting DNA fragment was ligated to pTrp4 that had previously been treated with NdeI/EcoRI. The solution containing the ligation product was used to transform *E*. *coli* JM109. Among the resulting ampicillin-resistant strains, a strain having the objective plasmid was selected, and the plasmid was designated pTrp4R (Fig. 6). In Figs. 6 and 7, the rmB terminator is referred to as "TrmB".

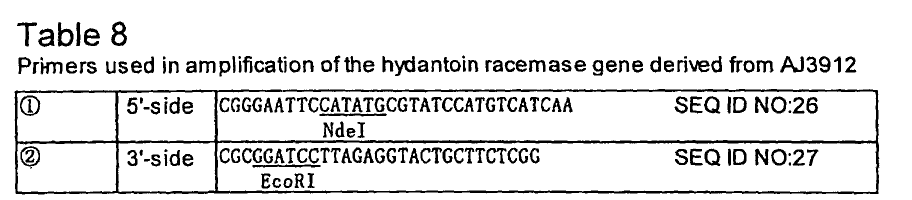

### 1-3. Construction of a plasmid carrying a D-hydantoinase gene and D-carbamylase gene

The objective genes were amplified by PCR with pTrpHrCr as a template and the oligonucleotides shown in Table 9 as primers. This fragment was treated with Ndel/EcoRI, and the resulting DNA fragment was ligated to pTrp8 that had previously been treated with Ndel/EcoRI. The solution containing the ligation product was used to transform *E*. *coli* JM109. Among the resulting chloramphenicol-resistant strains, a strain having the objective plasmid was selected, and the plasmid was designated pTrp8CH (Fig. 7).

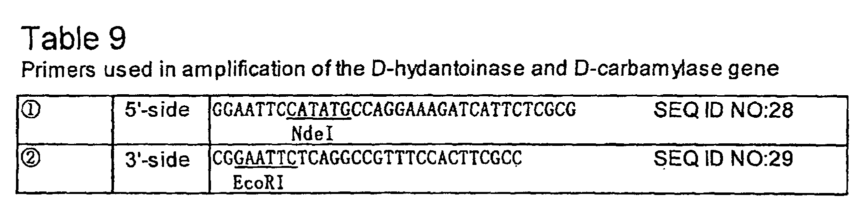

### 1-4. Preparation of a strain co-expressing the D-hydantoinase gene, D-carbamylase gene and hydantoin racemase gene, and production of D-Phe

The *E. coli* JM109 that already had pTrp4R was transformed by introducing pTrp8CH thereinto, to prepare a transformant having these two plasmids. This transformant was cultured at 30°C for 16 hours in LB medium (50 ml) containing 100 µg/ml ampicillin and 50 µg/ml chloramphenicol. 1 ml of the cultured liquid thus obtained was transferred to 300 ml of medium IV (2.5 percent glucose, 0.5 percent ammonium sulfate, 0.14 percent monopotassium dihydrogen phosphate, 0.23 percent trisodium citrate dihydrate, 0.002 percent iron(II) sulfate·heptahydrate, 0.1 percent magnesium sulfate·heptahydrate, 0.002 percent manganese sulfate·pentahydrate, 0.0001 percent thiamine hydrochloride, 0.01 percent ampicillin, 0.005 percent chloramphenicol (pH 7.0)), and cultured at 33°C for 24 hours in a jar fermenter while the pH was regulated at 7.0 and the dissolved oxygen concentration was regulated at 1.5 ppm or more. 15 ml of the cultured liquid thus obtained was transferred to 300 ml of medium V (2.5 percent glucose, 0.5 percent ammonium sulfate, 0.3 percent phosphoric acid, 0.23 percent trisodium citrate dihydrate, 0.1 percent magnesium sulfate·heptahydrate, 0.002 percent iron(II) sulfate·heptahydrate, 0.002 percent manganese sulfate·pentahydrate, 0.0001 percent thiamine hydrochloride, 0.01 percent ampicillin, 0.005 percent chloramphenicol (pH 7.0)) and cultured at 35°C for 24 hours in a jar fermenter while the pH was regulated at 7.0, and the dissolved oxygen concentration was regulated at 1.5 ppm or more. After the lapse of 9 hours from the beginning of the cultivation, 50 percent aqueous glucose solution was added thereto at the addition rate of 3.5 ml/hr. 15 ml of the cultured liquid (600 mg as the dry weight of the microorganism) thus obtained was added to 285 ml of a substrate solution (5.3 g/dl DL-5-benzyl hydantoin, 1.05 mM manganese sulfate, 21 mM KPB (pH 7.5)) and reacted at 37°C for 48 hours while the pH of the reaction liquid was kept at pH 7.5 (adjusted with 1 N NaOH and 2 N H₂SO₄). As a result, it was recognized that 4.15 g/dl (molar yield, 95 percent) of D-Phe was produced.

### 1-5. Production of D-Phe with the strain co-expressing the D-hydantoinase gene, D-carbamylase gene and hydantoin racemase gene (antibiotic-free system)

The *E*. *coli* JM109 having the two plasmids, i.e. pTrp4R and pTrp8CH, was cultured at 30°C for 16 hours in LM medium (50 ml) that was free of ampicillin and chloramphenicol. 1 ml of the cultured liquid thus obtained was transferred to 300 ml of medium IV that was free of ampicillin and chloramphenicol and cultured at 33°C for 24 hours in a jar fermenter while the pH was regulated at 7.0 and the dissolved oxygen concentration was regulated at 1.5 ppm or more. 15 ml of the cultured liquid thus obtained was transferred to 300 ml of medium V that was free of ampicillin and chloramphenicol and cultured at 35°C for 24 hours in a jar fermenter while the pH was regulated at 7.0 and the dissolved oxygen concentration was regulated at 1.5 ppm or more. After the lapse of 9 hours from the beginning of the cultivation, 50 percent aqueous glucose solution was added thereto at the addition rate of 3.5 ml/hr. 15 ml of the cultured liquid (600 mg as the dry weight of the microorganism) thus obtained was added to 285 ml of a substrate solution (5.3 g/dl DL-5-benzylhydantoin, 1.05 mM manganese sulfate, 21 mM KPB (pH 7.5)) and reacted at 37°C for 48 hours while the pH of the reaction liquid was kept at pH 7.5 (adjusted with 1 N NaOH and 2 N H₂SO₄). As a result, it was found that this antibiotic-free cultivation did not result in disappearance of the plasmids, and that 4.15 g/dl (molar yield, 95 percent) of D-Phe was produced.

### INDUSTRIAL APPLICABILITY

According to the present invention, there is provided a recombinant DNA co-expressing a hydantoinase gene and a carbamylase gene efficiently. According to the present invention, amino acids can be produced with high productivity.

| | | | |
|---|---|---|---|
| Name | Accession | Dates of original | Name and address of the |
| | Number | deposition and transfer | depositary authority |
| | | to the international | |
| | | deposition | |
| *Agrobacterium* | FERM | Date of original | Name: International Patent |
| sp. AJ 11220 | BP-7645 | deposition: December | Organism Depositary, |
| | | 20, 1977 | National Institute of |
| | | Date of transfer to the | Advanced Industrial |
| | | international deposition: | Science and Technology |
| | | June 27, 2001 | Address: Tsukuba Central |
| | | | 6,1-1-1 Higashi, Tsukuba, |
| | | | lbaraki, Japan |
| *Microbacterium* | FERM | Date of original | Name: International Patent |
| *liquefaciens* AJ | BP-7643 | deposition: June 27, | Organism Depositary, |
| 3912 | | 1975 | National Institute of |
| | | Date of transfer to the | Advanced Industrial |
| | | international deposition: | Science and Technology |
| | | June 27, 2001 | Address: Tsukuba Central |
| | | | 6,1-1-1 Higashi, Tsukuba, |
| | | | Ibaraki, Japan |
| *Flavobacterium* | FERM | Date of original | Name: International Patent |
| sp. AJ 11199 | BP-8063 | deposition: May 1, 1981 | Organism Depositary, |
| | | Date of transfer to the | National Institute of |
| | | international deposition: | Advanced Industrial |
| | | May 30, 2002 | Science and Technology |
| | | | Address: Tsukuba Central |
| | | | 6, 1-1-1 Higashi, Tsukuba, |
| | | | Ibaraki, Japan |

## Claims

1. A recombinant DNA comprising a base sequence encoding the following gene region (I), and another base sequence encoding the following gene region (II):
(I) a gene region containing a base sequence encoding a hydantoinase gene, and a trp promoter sequence located upstream of said base sequence encoding said hydantoinase gene, said trp promoter regulating expression of said hydantoinase gene;
(II) a gene region containing a base sequence encoding a carbamylase gene, and a trp promoter sequence located upstream of said base sequence encoding said carbamylase gene, said trp promoter regulating expression of said carbamylase gene.

2. The recombinant DNA according to claim 1, wherein the transcriptional direction of said base sequence encoding said gene region (I) is the same as that of said base sequence encoding said gene region (II), and wherein said base sequence encoding said gene region (I) and said base sequence encoding said gene region (II) are disposed in this order along said transcriptional direction.

3. The recombinant DNA according to claim 1, wherein the transcriptional direction of said base sequence encoding said gene region (I) is in the reverse direction of said base sequence encoding said gene region (II).

4. The recombinant DNA according to claim 1, wherein the transcriptional direction of said base sequence encoding said gene region (I) is the same as that of said base sequence encoding said gene region (II), and wherein said base sequence encoding said gene region (II) and said base sequence encoding said gene region (I) are disposed in this order along said transcriptional direction.

5. The recombinant DNA according to any one of claims 1 to 4, wherein said recombinant DNA is a plasmid.

6. A transformed cell transformed with said recombinant DNA according to any one of claims 1 to 5.

7. A transformed cell transformed with:
the recombinant DNA according to any one of claims 1 to 5; and
a recombinant DNA having a base sequence encoding a hydantoin racemase gene and a trp promoter sequence located upstream of said hydantoin racemase gene, said trp promoter regulating expression of said hydantoin racemase gene.

8. The transformed cell according to claim 7, wherein said recombinant DNA according to any one of claims 1 to 5 is a low-copy recombinant DNA, and said recombinant DNA having said base sequence encoding said hydantoin racemase gene and said trp promoter sequence located upstream of said hydantoin racemase gene regulating expression of said hydantoin racemase gene is a high-copy recombinant DNA.

9. The transformed cell according to any one of claims 6 to 8, wherein the host of said recombinant DNA is *Escherichia coli*.

10. A process for producing a mixture of proteins comprising the step of culturing the transformed cell according to claim 6 in a medium, to accumulate a protein having a hydantoinase activity and a protein having a carbamylase activity in said medium and/or said transformed cell.

11. A process for producing a mixture of proteins comprising the step of culturing said transformed cell according to claim 7 in a medium, to accumulate a protein having a hydantoinase activity, a protein having a carbamylase activity and a protein having a hydantoin racemase activity in said medium and/or said transformed cell.

12. A process for producing an amino acid comprising the step of culturing the transformed cell according to any one of claims 6 to 9 in a medium to give a culture, and the step of mixing 5-substituted hydantoin with said culture to form said amino acid.


SEQUENCE LISTING
<110> Ajinomoto Co., Inc.
<120> Recombinant DNA having hydantoinase gene and carbamylase gene and process for producing amino acid
<130> EPA-63542
<150> JP2002-108550
<151> 2002-04-10
<160> 29
<210> 1
<211> 1458
<212> DNA
<213> Flavobacterium sp.
<220>
<221> CDS
<222> (1)..(1455)
<223> DHHase
<400> 1
atg acc cat tac gat ctc gtc att cgc gga gga acc gtc gcc acg gcg 48
Met Thr His Tyr Asp Leu Val Ile Arg Gly Gly Thr Val Ala Thr Ala
1               5                   10                  15
agt gat tgc ttc cgg gcg gat gtt gcg gtc acc gac ggc agg atc gtc 96
Ser Asp Cys Phe Arg Ala Asp Val Ala Val Thr Asp Gly Arg Ile Val
            20                  25                  30
gcc atc ggc gag aat ctc ggg ccc gcc gcc cgc gag atc tcg gcg gaa 144
Ala Ile Gly Glu Asn Leu Gly Pro Ala Ala Arg Glu Ile Ser Ala Glu
        35                  40                  45
ggc cgc ctc gtt ctg ccc ggc ggc gtc gat tcc cat tgc cat atc gag 192
Gly Arg Leu Val Leu Pro Gly Gly Val Asp Ser His Cys His Ile Glu
    50                  55                  60
gag ccg cag gtc ggc gag gtg cgc aac gcg gaa acc ttc gcc tcc gcc 240
Glu Pro Gln Val Gly Glu Val Arg Asn Ala Glu Thr Phe Ala Ser Ala
65                  70                  75                  80
acc tct tcg gcg gcc gcg ggc ggc acg acg acg gtc att tcc ttc tct 288
Thr Ser Ser Ala Ala Ala Gly Gly Thr Thr Thr Val Ile Ser Phe Ser
                85                  90                  95
cag cag gtg aaa ggc ggc ggc atc acc gag gcc ctg cgc gac tat cac 336
Gln Gln Val Lys Gly Gly Gly Ile Thr Glu Ala Leu Arg Asp Tyr His
            100                 105                 110
gag aag gcg acc cgc gcg ctg atc gac tat tcc ttc cat ctc gtc gtg 384
Glu Lys Ala Thr Arg Ala Leu Ile Asp Tyr Ser Phe His Leu Val Val
        115                 120                 125
acc gat ccc acg gat gcg gtg ctg gag gaa ctg gtg ccg ctg atc gag 432
Thr Asp Pro Thr Asp Ala Val Leu Glu Glu Leu Val Pro Leu Ile Glu
    130                 135                 140
gaa ggg cac cgg tcg ctg aag atc ttc atg acc tat acc aat gtc gtg 480
Glu Gly His Arg Ser Leu Lys Ile Phe Met Thr Tyr Thr Asn Val Val
145                 150                 155                 160
ctc gac gac gag cag acg ctg cgc gtg ctg gcg ctc gcc cgc aag acc 528
Leu Asp Asp Glu Gln Thr Leu Arg Val Leu Ala Leu Ala Arg Lys Thr
                165                 170                 175
ggc gct ctc gtc acg gtg cat gcc gag aac cat gcc gcg atc atg tac 576
Gly Ala Leu Val Thr Val His Ala Glu Asn His Ala Ala Ile Met Tyr
            180                 185                 190
ctc acg cgt gcc ttg gaa aag gcc ggg ctc acc gcc ccg aag tac cac 624
Leu Thr Arg Ala Leu Glu Lys Ala Gly Leu Thr Ala Pro Lys Tyr His
        195                 200                 205
gcc tgg gcc aag ccc atg ccg gtg gaa cgg gag gcc tgc cac cgc atc 672
Ala Trp Ala Lys Pro Met Pro Val Glu Arg Glu Ala Cys His Arg Ile
    210                 215                 220
atc atg ctc tcc gaa ctg ctg gat gtt ccc att cag atc ttc cac gtt 720
Ile Met Leu Ser Glu Leu Leu Asp Val Pro Ile Gln Ile Phe His Val
225                 230                 235                 240
tcc ggg gcg gag gcg gcc gag gaa atc cgc cgc gcg cag aat cgc ggc 768
Ser Gly Ala Glu Ala Ala Glu Glu Ile Arg Arg Ala Gln Asn Arg Gly
                245                 250                 255
ctc aag gtc ttc ggc gag acc tgc ccg caa tat ctg atg ctg acg gcg 816
Leu Lys Val Phe Gly Glu Thr Cys Pro Gln Tyr Leu Met Leu Thr Ala
            260                 265                 270
gcg gat ctc gac cgg ccg gat ttc gag ggt gcg aaa ttc ctg tgc agc 864
Ala Asp Leu Asp Arg Pro Asp Phe Glu Gly Ala Lys Phe Leu Cys Ser
        275                 280                 285
ccc gcg ccg cgc acg acg gcc gat cag gaa gcc ctg tgg gac tat atc 912
Pro Ala Pro Arg Thr Thr Ala Asp Gln Glu Ala Leu Trp Asp Tyr Ile
    290                 295                 300
cgc acg gga acg atc ggg gtc gtg tcg tcc gac cac gcg ccg aac cgc 960
Arg Thr Gly Thr Ile Gly Val Val Ser Ser Asp His Ala Pro Asn Arg

305                 310                 315                 320
ttc gac gat ccg cac ggc aag aag gtc ggc ggg gtg gac gcg ccc ttc     1008
Phe Asp Asp Pro His Gly Lys Lys Val Gly Gly Val Asp Ala Pro Phe
                325                 330                 335
agc gcc att ccg aac ggc gtg ccg ggg ctg gcg acg cgc ctg ccg atc     1056
Ser Ala Ile Pro Asn Gly Val Pro Gly Leu Ala Thr Arg Leu Pro Ile
            340                 345                 350
ctg ttt tcc gaa ggg gtc gtc aag ggg cgc atc gac ctc aac acc ttc     1104
Leu Phe Ser Glu Gly Val Val Lys Gly Arg Ile Asp Leu Asn Thr Phe
        355                 360                 365
gtc gcg ctg acg gcg gcc aat ccg gcg aag ctc ttt ggc ctg cat ccc     1152
Val Ala Leu Thr Ala Ala Asn Pro Ala Lys Leu Phe Gly Leu His Pro
    370                 375                 380
aga aag ggc acc atc gcc gtc gga gcc gat gcg gat atc gcg atc tgg     1200
Arg Lys Gly Thr Ile Ala Val Gly Ala Asp Ala Asp Ile Ala Ile Trp
385                 390                 395                 400
gat ccc gag cgc aag gtg acg atc cgc aac gac atg ctg cat cac ggc     1248
Asp Pro Glu Arg Lys Val Thr Ile Arg Asn Asp Met Leu His His Gly
                405                 410                 415
gtc gac tac acg gtc tac gag ggc gtc gag gtg acc gga tgg ccg gtg     1296
Val Asp Tyr Thr Val Tyr Glu Gly Val Glu Val Thr Gly Trp Pro Val
            420                 425                 430
atg acg ctc tcg cgc ggc gag gtg gtc tat gat gac ggc gcg gtg gtc     1344
Met Thr Leu Ser Arg Gly Glu Val Val Tyr Asp Asp Gly Ala Val Val
        435                 440                 445
ggc aag ccg ggt cac ggg cgc ttc ctt gcc cgc ggt ccc tat gac gcg     1392
Gly Lys Pro Gly His Gly Arg Phe Leu Ala Arg Gly Pro Tyr Asp Ala
    450                 455                 460
atc aag ccg cgc ggc agg cat gtg acg cct ttc gac ccg gtg acg ggc     1440
Ile Lys Pro Arg Gly Arg His Val Thr Pro Phe Asp Pro Val Thr Gly
465                 470                 475                 480
gaa gtg gaa acg gcc tga                                             1458
Glu Val Glu Thr Ala
                485
<210> 2
<211> 485
<212> PRT
<213> Flavobacterium sp.
<400> 2
Met Thr His Tyr Asp Leu Val Ile Arg Gly Gly Thr Val Ala Thr Ala
 1               5                  10                  15
Ser Asp Cys Phe Arg Ala Asp Val Ala Val Thr Asp Gly Arg Ile Val
            20                  25                  30
Ala Ile Gly Glu Asn Leu Gly Pro Ala Ala Arg Glu Ile Ser Ala Glu
        35                  40                  45
Gly Arg Leu Val Leu Pro Gly Gly Val Asp Ser His Cys His Ile Glu
    50                  55                  60
Glu Pro Gln Val Gly Glu Val Arg Asn Ala Glu Thr Phe Ala Ser Ala
65                  70                  75                  80
Thr Ser Ser Ala Ala Ala Gly Gly Thr Thr Thr Val Ile Ser Phe Ser
                85                  90                  95
Gln Gln Val Lys Gly Gly Gly Ile Thr Glu Ala Leu Arg Asp Tyr His
            100                 105                 110
Glu Lys Ala Thr Arg Ala Leu Ile Asp Tyr Ser Phe His Leu Val Val
        115                 120                 125
Thr Asp Pro Thr Asp Ala Val Leu Glu Glu Leu Val Pro Leu Ile Glu
    130                 135                 140
Glu Gly His Arg Ser Leu Lys Ile Phe Met Thr Tyr Thr Asn Val Val
145                 150                 155                 160
Leu Asp Asp Glu Gln Thr Leu Arg Val Leu Ala Leu Ala Arg Lys Thr
                165                 170                 175
Gly Ala Leu Val Thr Val His Ala Glu Asn His Ala Ala Ile Met Tyr
            180                 185                 190
Leu Thr Arg Ala Leu Glu Lys Ala Gly Leu Thr Ala Pro Lys Tyr His
        195                 200                 205
Ala Trp Ala Lys Pro Met Pro Val Glu Arg Glu Ala Cys His Arg Ile
    210                 215                 220
Ile Met Leu Ser Glu Leu Leu Asp Val Pro Ile Gln Ile Phe His Val
225                 230                 235                 240
Ser Gly Ala Glu Ala Ala Glu Glu Ile Arg Arg Ala Gln Asn Arg Gly
                245                 250                 255
Leu Lys Val Phe Gly Glu Thr Cys Pro Gln Tyr Leu Met Leu Thr Ala
            260                 265                 270
Ala Asp Leu Asp Arg Pro Asp Phe Glu Gly Ala Lys Phe Leu Cys Ser
        275                 280                 285

Pro Ala Pro Arg Thr Thr Ala Asp Gln Glu Ala Leu Trp Asp Tyr Ile
    290                 295                 300
Arg Thr Gly Thr Ile Gly Val Val Ser Ser Asp His Ala Pro Asn Arg
305                 310                 315                 320
Phe Asp Asp Pro His Gly Lys Lys Val Gly Gly Val Asp Ala Pro Phe
                325                 330                 335
Ser Ala Ile Pro Asn Gly Val Pro Gly Leu Ala Thr Arg Leu Pro Ile
            340                 345                 350
Leu Phe Ser Glu Gly Val Val Lys Gly Arg Ile Asp Leu Asn Thr Phe
        355                 360                 365
Val Ala Leu Thr Ala Ala Asn Pro Ala Lys Leu Phe Gly Leu His Pro
    370                 375                 380
Arg Lys Gly Thr Ile Ala Val Gly Ala Asp Ala Asp Ile Ala Ile Trp
385                 390                 395                 400
Asp Pro Glu Arg Lys Val Thr Ile Arg Asn Asp Met Leu His His Gly
                405                 410                 415
Val Asp Tyr Thr Val Tyr Glu Gly Val Glu Val Thr Gly Trp Pro Val
            420                 425                 430
Met Thr Leu Ser Arg Gly Glu Val Val Tyr Asp Asp Gly Ala Val Val
        435                 440                 445
Gly Lys Pro Gly His Gly Arg Phe Leu Ala Arg Gly Pro Tyr Asp Ala
    450                 455                 460
Ile Lys Pro Arg Gly Arg His Val Thr Pro Phe Asp Pro Val Thr Gly
465                 470                 475                 480
Glu Val Glu Thr Ala
                485
<210> 3
<211> 915
<212> DNA
<213> Flavobacterium sp.
<220>
<221> CDS
<222> (1)..(912)
<223> DCHase
<400> 3
atg cca gga aag atc att ctc gcg gtg ggc cag ctt gga ccc atc gcc 48
Met Pro Gly Lys Ile Ile Leu Ala Val Gly Gln Leu Gly Pro Ile Ala
1               5                   10                  15
cgc agc gac agc cgc gaa cag gtg gtg aag cgg ctc gtc gac atg ctg 96
Arg Ser Asp Ser Arg Glu Gln Val Val Lys Arg Leu Val Asp Met Leu
            20                  25                  30
gag gag gcc gcg gcc cgc aat tcg gat ttc atc gtc ttt ccc gag ctg 144
Glu Glu Ala Ala Ala Arg Asn Ser Asp Phe Ile Val Phe Pro Glu Leu
        35                  40                  45
gcg ctg acc acc ttc ttc ccc cgc tgg tat ttc acc gac gag gcg gaa 192
Ala Leu Thr Thr Phe Phe Pro Arg Trp Tyr Phe Thr Asp Glu Ala Glu
    50                  55                  60
ctg gac gcg ttc tac gag acg gaa atg ccg agc ccg gtg aca cgc ccg 240
Leu Asp Ala Phe Tyr Glu Thr Glu Met Pro Ser Pro Val Thr Arg Pro
65                  70                  75                  80
ctg ttc gag gcc gcc gcg aaa cac ggc gtc ggg ttc aat ctc ggc ttc 288
Leu Phe Glu Ala Ala Ala Lys His Gly Val Gly Phe Asn Leu Gly Phe
                85                  90                  95
gcg gag ctc gtg tcg gag ggc ggc agg aag cgc cgc ttc aac acg tcc 336
Ala Glu Leu Val Ser Glu Gly Gly Arg Lys Arg Arg Phe Asn Thr Ser
            100                 105                 110
atc gcc gtc gac agg acc ggc agg atc atc ggc aag tac cgc aag atc 384
Ile Ala Val Asp Arg Thr Gly Arg Ile Ile Gly Lys Tyr Arg Lys Ile
        115                 120                 125
cat ctg ccg ggc cac aag gac tat gaa gac tac cgc ccg ttc cag cat 432
His Leu Pro Gly His Lys Asp Tyr Glu Asp Tyr Arg Pro Phe Gln His
    130                 135                 140
ctg gag aag cgg tac ttc gag acc ggc aat ctc gga ttc ccg gtc tat 480
Leu Glu Lys Arg Tyr Phe Glu Thr Gly Asn Leu Gly Phe Pro Val Tyr
145                 150                 155                 160
gag gtc gat ggt ttc aag atg ggc atg ttc atc tgc aac gac cgg cgc 528
Glu Val Asp Gly Phe Lys Met Gly Met Phe Ile Cys Asn Asp Arg Arg
                165                 170                 175
tgg ccg gag acc tgg cgc gtg atg ggt ctc aag ggc gcg gag ctg atc 576
Trp Pro Glu Thr Trp Arg Val Met Gly Leu Lys Gly Ala Glu Leu Ile
            180                 185                 190
tgc ggc ggc tac aac acg ccg ctg cac aat ccg ccg gtg ccg cag cac 624
Cys Gly Gly Tyr Asn Thr Pro Leu His Asn Pro Pro Val Pro Gln His
        195                 200                 205
gac cag ctc agc tcc ttc cac cat ctc ctg tcg atg cag gcg ggc gcc 672

Asp Gln Leu Ser Ser Phe His His Leu Leu Ser Met Gln Ala Gly Ala
    210                 215                 220
tac cag aac ggt gcg tgg acc gcc gga gcg ggc aag gtc ggg gtg gag     720
Tyr Gln Asn Gly Ala Trp Thr Ala Gly Ala Gly Lys Val Gly Val Glu
225                 230                 235                 240
gaa ggc tgc atg ctg ctc ggc cat tcc tgc atc gtc gcg ccc acc ggg     768
Glu Gly Cys Met Leu Leu Gly His Ser Cys Ile Val Ala Pro Thr Gly
                245                 250                 255
gag ctt gcc gcg ctc acc aac acg ctg gag gac gag gtc atc acg gcc     816
Glu Leu Ala Ala Leu Thr Asn Thr Leu Glu Asp Glu Val Ile Thr Ala
            260                 265                 270
gct gcc gat ttt gcc cgc tgc cgg gaa atc cgc gag aac atc ttc aat     864
Ala Ala Asp Phe Ala Arg Cys Arg Glu Ile Arg Glu Asn Ile Phe Asn
        275                 280                 285
ttc gag cag cat cgc gag ccg cag gaa tac ggg ttg atc gcc gcc gtc     912
Phe Glu Gln His Arg Glu Pro Gln Glu Tyr Gly Leu Ile Ala Ala Val
    290                 295                 300
tga                                                                 915
<210> 4
<211> 304
<212> PRT
<213> Flavobacterium sp.
<400> 4
Met Pro Gly Lys Ile Ile Leu Ala Val Gly Gln Leu Gly Pro Ile Ala
  1               5                  10                  15
Arg Ser Asp Ser Arg Glu Gln Val Val Lys Arg Leu Val Asp Met Leu
             20                  25                  30
Glu Glu Ala Ala Ala Arg Asn Ser Asp Phe Ile Val Phe Pro Glu Leu
         35                  40                  45
Ala Leu Thr Thr Phe Phe Pro Arg Trp Tyr Phe Thr Asp Glu Ala Glu
     50                  55                  60
Leu Asp Ala Phe Tyr Glu Thr Glu Met Pro Ser Pro Val Thr Arg Pro
 65                  70                  75                  80
Leu Phe Glu Ala Ala Ala Lys His Gly Val Gly Phe Asn Leu Gly Phe
                 85                  90                  95
Ala Glu Leu Val Ser Glu Gly Gly Arg Lys Arg Arg Phe Asn Thr Ser
            100                 105                 110
Ile Ala Val Asp Arg Thr Gly Arg Ile Ile Gly Lys Tyr Arg Lys Ile
        115                 120                 125
His Leu Pro Gly His Lys Asp Tyr Glu Asp Tyr Arg Pro Phe Gln His
    130                 135                 140
Leu Glu Lys Arg Tyr Phe Glu Thr Gly Asn Leu Gly Phe Pro Val Tyr
145                 150                 155                 160
Glu Val Asp Gly Phe Lys Met Gly Met Phe Ile Cys Asn Asp Arg Arg
                165                 170                 175
Trp Pro Glu Thr Trp Arg Val Met Gly Leu Lys Gly Ala Glu Leu Ile
            180                 185                 190
Cys Gly Gly Tyr Asn Thr Pro Leu His Asn Pro Pro Val Pro Gln His
        195                 200                 205
Asp Gln Leu Ser Ser Phe His His Leu Leu Ser Met Gln Ala Gly Ala
    210                 215                 220
Tyr Gln Asn Gly Ala Trp Thr Ala Gly Ala Gly Lys Val Gly Val Glu
225                 230                 235                 240
Glu Gly Cys Met Leu Leu Gly His Ser Cys Ile Val Ala Pro Thr Gly
                245                 250                 255
Glu Leu Ala Ala Leu Thr Asn Thr Leu Glu Asp Glu Val Ile Thr Ala
            260                 265                 270
Ala Ala Asp Phe Ala Arg Cys Arg Glu Ile Arg Glu Asn Ile Phe Asn
        275                 280                 285
Phe Glu Gln His Arg Glu Pro Gln Glu Tyr Gly Leu Ile Ala Ala Val
    290                 295                 300
<210> 5
<211> 711
<212> DNA
<213> Microbacterium liquefaciens
<220>
<221> CDS
<222> (1)..(708)
<223> HRase
<400> 5
atg aga atc cat gtc atc aat ccc aac agc tcg gtg gat ctc acc gat     48
Met Arg Ile His Val Ile Asn Pro Asn Ser Ser Val Asp Leu Thr Asp
  1               5                  10                  15
gcg gtg gcc gag gcg gcg cga agt gtg gtg tca ccg gga acc acc atc     96
Ala Val Ala Glu Ala Ala Arg Ser Val Val Ser Pro Gly Thr Thr Ile

                20                  25                  30
acc gcg gtc aac cct tcg aag ggc ccc acg gtc atc gag ggc agt tac    144
Thr Ala Val Asn Pro Ser Lys Gly Pro Thr Val Ile Glu Gly Ser Tyr
            35                  40                  45
gac gag gtg ctg gcc acg tat cac ctc gtc gaa gag gtc cgc cgc gcg    192
Asp Glu Val Leu Ala Thr Tyr His Leu Val Glu Glu Val Arg Arg Ala
    50                  55                  60
gag cgc gaa gac cga ccg gac gcc tac gtc atc gcc tgt ttc ggc gat    240
Glu Arg Glu Asp Arg Pro Asp Ala Tyr Val Ile Ala Cys Phe Gly Asp
65                  70                  75                  80
cca ggt ctc gac gcc gtc agg gag ctc acc gac agg ccc gtg gtc gga    288
Pro Gly Leu Asp Ala Val Arg Glu Leu Thr Asp Arg Pro Val Val Gly
                85                  90                  95
atc gcc gag gcg gcg atc cag atg acg agc ttc gtc gcc gcg agc ttc    336
Ile Ala Glu Ala Ala Ile Gln Met Thr Ser Phe Val Ala Ala Ser Phe
            100                 105                 110
tcc atc gtg agc atc ctc ccg cgc gtg cgc aag cat ctg cac gag ctg    384
Ser Ile Val Ser Ile Leu Pro Arg Val Arg Lys His Leu His Glu Leu
        115                 120                 125
gtg cac cgg gcg ggg gca acg gat cga ctc gcc tca ctc aag ctt ccg    432
Val His Arg Ala Gly Ala Thr Asp Arg Leu Ala Ser Leu Lys Leu Pro
    130                 135                 140
gat ctc gga gtg ctc gca ttc cac gag gac gag gca gcg gcg ttc gag    480
Asp Leu Gly Val Leu Ala Phe His Glu Asp Glu Ala Ala Ala Phe Glu
145                 150                 155                 160
acc ctc cgg cgc gtg gca ggt gag gcg gtg cgc gag gac ggc gcg gag    528
Thr Leu Arg Arg Val Ala Gly Glu Ala Val Arg Glu Asp Gly Ala Glu
                165                 170                 175
tcg atc gtg ctc ggc tgc gcg ggc atg gcc gga ttc gcc aga cag ctg    576
Ser Ile Val Leu Gly Cys Ala Gly Met Ala Gly Phe Ala Arg Gln Leu
            180                 185                 190
agt gaa gag ctc ggc gtc ccc gtc atc gac gcg gtc gag gca gcc tgc    624
Ser Glu Glu Leu Gly Val Pro Val Ile Asp Ala Val Glu Ala Ala Cys
        195                 200                 205
cgc gtc gcg gag agc ctc gtc gcc ctg ggg tac cgc acc agc aag gcg    672
Arg Val Ala Glu Ser Leu Val Ala Leu Gly Tyr Arg Thr Ser Lys Ala
    210                 215                 220
aac acc tac caa gca ccc acc gag aag cag tac ctc taa                711
Asn Thr Tyr Gln Ala Pro Thr Glu Lys Gln Tyr Leu
225                 230                 235
<210> 6
<211> 236
<212> PRT
<213> Microbacterium liquefaciens
<400> 6
Met Arg Ile His Val Ile Asn Pro Asn Ser Ser Val Asp Leu Thr Asp
1               5                   10                  15
Ala Val Ala Glu Ala Ala Arg Ser Val Val Ser Pro Gly Thr Thr Ile
            20                  25                  30
Thr Ala Val Asn Pro Ser Lys Gly Pro Thr Val Ile Glu Gly Ser Tyr
        35                  40                  45
Asp Glu Val Leu Ala Thr Tyr His Leu Val Glu Glu Val Arg Arg Ala
    50                  55                  60
Glu Arg Glu Asp Arg Pro Asp Ala Tyr Val Ile Ala Cys Phe Gly Asp
65                  70                  75                  80
Pro Gly Leu Asp Ala Val Arg Glu Leu Thr Asp Arg Pro Val Val Gly
                85                  90                  95
Ile Ala Glu Ala Ala Ile Gln Met Thr Ser Phe Val Ala Ala Ser Phe
            100                 105                 110
Ser Ile Val Ser Ile Leu Pro Arg Val Arg Lys His Leu His Glu Leu
        115                 120                 125
Val His Arg Ala Gly Ala Thr Asp Arg Leu Ala Ser Leu Lys Leu Pro
    130                 135                 140
Asp Leu Gly Val Leu Ala Phe His Glu Asp Glu Ala Ala Ala Phe Glu
145                 150                 155                 160
Thr Leu Arg Arg Val Ala Gly Glu Ala Val Arg Glu Asp Gly Ala Glu
                165                 170                 175
Ser Ile Val Leu Gly Cys Ala Gly Met Ala Gly Phe Ala Arg Gln Leu
            180                 185                 190
Ser Glu Glu Leu Gly Val Pro Val Ile Asp Ala Val Glu Ala Ala Cys
        195                 200                 205
Arg Val Ala Glu Ser Leu Val Ala Leu Gly Tyr Arg Thr Ser Lys Ala
    210                 215                 220
Asn Thr Tyr Gln Ala Pro Thr Glu Lys Gln Tyr Leu

225                    230                    235
<210> 7
<211> 26
<212> DNA
<213> Artificial Sequence
<220>
<223> Description of Artificial Sequence:primer(7)
<400> 7
ggggtaccgg agctgatacc atgacc                                          26
<210> 8
<211> 26
<212> DNA
<213> Artificial Sequence
<220>
<223> Description of Artificial Sequence:primer(8)
<400> 8
ggggtaccgc aaccgatagc cggagc                                          26
<210> 9
<211> 26
<212> DNA
<213> Artificial Sequence
<220>
<223> Description of Artificial Sequence:primer (9)
<400> 9
ggggtaccat gacccattac gatctc                                          26
<210> 10
<211> 26
<212> DNA
<213> Artificial Sequence
<220>
<223> Description of Artificial Sequence:primer(10)
<400> 10
ggggtaccac catgacccat tacgat                                          26
<210> 11
<211> 26
<212> DNA
<213> Artificial Sequence
<220>
<223> Description of Artificial Sequence:primer(11)
<400> 11
gctctagacg tcctgtcctt tccgcc                                          26
<210> 12
<211> 26
<212> DNA
<213> Artificial Sequence
<220>
<223> Description of Artificial Sequence:primer(12)
<400> 12
ggggtaccgg agaggaatat gccagg                                          26
<210> 13
<211> 27
<212> DNA
<213> Artificial Sequence
<220>
<223> Description of Artificial Sequence:primer(13)
<400> 13
ggggtaccgg gaaaggaaaa ggagagg                                         27
<210> 14
<211> 26
<212> DNA
<213> Artificial Sequence
<220>
<223> Description of Artificial Sequence:primer(14)
<400> 14
gctctagagc cgcgccgctc agacgg                                          26
<210> 15
<211> 26
<212> DNA
<213> Artificial Sequence
<220>
<223> Description of Artificial Sequence:primer(15)
<400> 15
gctctagaat gagaatccat gtcatc                                          26
<210> 16
<211> 26

<212> DNA
<213> Artificial Sequence
<220>
<223> Description of Artificial Sequence:primer(16)
<400> 16
gctctagagc gatgcgtatc catgtc 26
<210> 17
<211> 26
<212> DNA
<213> Artificial Sequence
<220>
<223> Description of Artificial Sequence:primer(17)
<400> 17
ggctgcagcg ctccttctcg ttagag 26
<210> 18
<211> 25
<212> DNA
<213> Artificial Sequence
<220>
<223> Description of Artificial Sequence:primer(18)
<400> 18
cgtctagatg ttgacaatta atcat 25
<210> 19
<211> 32
<212> DNA
<213> Artificial Sequence
<220>
<223> Description of Artificial Sequence:primer(19)
<400> 19
cgctgcagtc agacggcggc gatcaacccg ta 32
<210> 20
<211> 32
<212> DNA
<213> Artificial Sequence
<220>
<223> Description of Artificial Sequence:primer(20)
<400> 20
cgctgcagtc aggccgtttc cacttcgccc gt 32
<210> 21
<211> 67
<212> DNA
<213> Artificial Sequence
<220>
<223> Description of Artificial Sequence:a sequence of
cassette 1 including trp promoter
<400> 21
aattctgttg acaataattc atcgaactag ttaactagta cgcaagttca cgtaaaaagg 60
gtggtac 67
<210> 22
<211> 63
<212> DNA
<213> Artificial Sequence
<220>
<223> Description of Artificial Sequence:a sequence of
cassette 2 including trp promoter
<400> 22
aattctgttg acaataattc atcgaactag ttaactagta cgcaagttca cgtaaaaagg 60
gtt 63
<210> 23
<211> 40
<212> DNA
<213> Artificial Sequence
<220>
<223> Description of Artificial Sequence:primer(23)
<400> 23
gtatcacgag gccctagctg tggtgtcatg gtcggtgatc 40
<210> 24
<211> 40
<212> DNA
<213> Artificial Sequence
<220>
<223> Description of Artificial Sequence:primer(24)
<400> 24
ttcggggatt ccatatgata ccctttttac gtgaacttgc 40
<210> 25

<211> 38
<212> DNA
<213> Artificial Sequence
<220>
<223> Description of Artificial Sequence:primer(25)
<400> 25
ggggggggca tatgcgacct ccttattacg tgaacttg 38
<210> 26
<211> 32
<212> DNA
<213> Artificial Sequence
<220>
<223> Description of Artificial Sequence:primer(26)
<400> 26
cgggaattcc atatgcgtat ccatgtcatc aa 32
<210> 27
<211> 29
<212> DNA
<213> Artificial Sequence
<220>
<223> Description of Artificial Sequence:primer(27)
<400> 27
cgcggatcct tagaggtact gcttctcgg 29
<210> 28
<211> 34
<212> DNA
<213> Artificial Sequence
<220>
<223> Description of Artificial Sequence:primer(28)
<400> 28
ggaattccat atgccaggaa agatcattct cgcg 34
<210> 29
<211> 29
<212> DNA
<213> Artificial Sequence
<220>
<223> Description of Artificial Sequence:primer(29)
<400> 29
cggaattctc aggccgtttc cacttcgcc 29